# EUROPEAN PATENT APPLICATION

(11) **EP 1 719 746 A1**
(43) Date of publication of application: **08.11.2006**
(21) Application number: 05710698.1
(22) Date of filing: 18.02.2005
(51) Int. Cl.: C07B 57/00, C07C 29/88, C07C 31/125, C07C 33/03, C07C 33/18, C07C 33/20, C07C 67/60, C07C 69/68, C07D 307/935

(54) **OPTICAL RESOLVER, PROCESS FOR PRODUCING OPTICALLY ACTIVE ISOMER, AND 1,5-SUBSTITUTED BICYCLO (3.3.0) -2-OXAOCTANE COMPOUND**

(30) Priority: 19.02.2004 JP 2004077186
(71) Applicant: ZEON CORPORATION, Tokyo 100-8246 (JP)
(72) Inventor: NEMOTO, Hisao, Tokushima-shi, Tokushima 7708079 (JP)
(74) Representative: Albrecht, Thomas
(86) International application number: PCT/JP2005/003098
(87) International publication number: WO 2005/080300

(57) **Abstract**

An agent for optical resolution comprising a bicyclo [3.3.0] -2-oxaoctane compound represented by the formula [1], [2] or [3] ; a process for producing an optically active substance comprising obtaining a mixture of diastereomers by reaction of the agent with a mixture of optically active substances having active hydrogen atom, resolving the mixture of diastereomers into each diastereomer and obtaining an (R) optically active substance or an (S) optically active substance by decomposition of the diastereomer; and compounds represented by the above formulae in which R¹¹ represents fluorenylmethyl group, fluorenylidenemethyl group, bis(4-cyclohexylphenyl)methyl group, 4-(9-phenanthryl)phenyl group, 4-(1-pyrenyl)phenyl group, 4-(5-acenaphthenyl)phenyl group or 4-(9-anthryl)phenyl group, are disclosed. A useful optically active substance having a very high purity can be produced efficiently by using the agent of the present invention.

## Description

### TECHNICAL FIELD

The present invention relates to an agent for optical resolution, a process for producing an optically active substance and an optically active substance. More particularly, the present invention relates to an agent for optical resolution and a process for producing an optically active substance which can efficiently produce a useful optically active substance having a very high optical purity, and a novel compound having a substituent having a fluorene structure and useful as an agent for optical resolution.

### BACKGROUND ART

Many physiologically active substances such as drugs, agricultural chemicals, perfumes and seasonings are optically active substances having asymmetric carbon atom and active hydrogen atom or derivatives thereof. An optically active substance has optical isomers. The strength of the physiological activity is occasionally greatly different between the optical isomers and, in some cases, entirely different physiological activities are exhibited by the optical isomers. Therefore, a process for optical resolution which allows easy and sure resolution of optical isomers having asymmetric carbon atom and active hydrogen atom from a mixture thereof to produce an optically active substance having a very high optical purity is desired.

As an example of optical resolution of an alcohol, a process in which optical resolution is achieved by esterifying one of optical isomers utilizing a natural environment of optical activity such as an organ containing an esterification enzyme or a hydrolysis enzyme while the other optical isomer is left remaining as the alcohol, is reported (Non-Patent Reference 1 and Non-Patent Reference 2). However, an enzyme such as those described above cannot be used at high temperatures since the enzyme has poor chemical stability and, in particular, poor stability under heating, and it is difficult that the enzyme is obtained in a great amount. Therefore, the above process has a drawback in that the process cannot be used widely for many types of substances.

A case in which a carboxylic acid having an asymmetric carbon atom and an alcohol are condensed to form an ester, and diastereomers are resolved in accordance with a silica gel column chromatography to obtain each diastereomer, is reported (Non-Patent Reference 3). This process can be considered to be a process for optical resolution of an alcohol. However, this process has a drawback in that there is no general rule or principle on formation of a mixture of diastereomers allowing resolution to a great degree, and the process cannot be used widely for many types of substances.

The cases in which optically active substances are resolved without providing an optically active external factor, such as spontaneous resolution, are rarely found. No general rule for the resolution is existing. Therefore, in most cases, it is very difficult to predict whether optical resolution of a mixture of optically active substances having active hydrogen atom is possible or not.

In Patent Reference 1, an excellent agent for optical resolution having a bicyclooxaoctane ring is disclosed. In Patent Reference 1, bicyclooxaoctane rings having an alkyl group, an alkenyl group, formyl group or an acyl group are examined. An agent for optical resolution exhibiting more excellent ability of resolution has been desired.
(Non-Patent Reference 1) Synlett., (66), 862 (2000)
(Non-Patent Reference 2) J. Org. Chem., (64), 2638 (1999)
(Non-Patent Reference 3) Tetrahedron Lett., (35), 4397 (1994)
(Patent Reference 1) WO 02/072505

The present invention has an object of providing an agent for optical resolution and a process for producing an optically active substance which can efficiently produce a useful optically active substance having a very high optical purity, and a novel compound having a substituent having a fluorene structure and useful as an agent for optical resolution.

### DISCLOSURE OF THE INVENTION

As the result of extensive studies by the present inventors to overcome the above problems, it was found that a 1-alkoxybicyclo [3.3.0] 2-oxaoctane having a condensed polycyclic hydrocarbon group or group having at least three cyclic structures at the 5-position reacted quantitatively with a mixture of optical active substances having active hydrogen atom to form a mixture of diastereomers having an optically active group at the 1-position, the mixture of diastereomers could be easily resolved, and the diastereomers obtained by the resolution could be easily decomposed to provide the (R) isomer or the (S) isomer of the optically active substance having a very high optical purity. The present invention has been completed based on the knowledge.

The present invention provides
(1) An agent for optical resolution which comprises:
   a 1-alkoxybicyclo [3.3.0] -2-oxaoctane compound represented by the formula [1],
   a 1-hydroxybicyclo [3.3.0] -2-oxaoctane compound represented by the formula [2] or
   a bicyclo [3.3.0] -2-oxa-1-octene compound represented by the formula [3]: 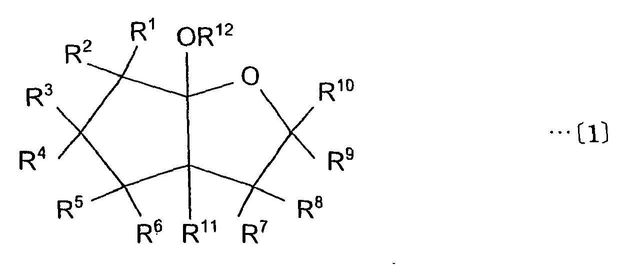 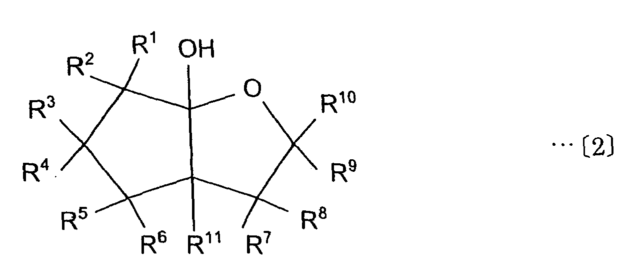 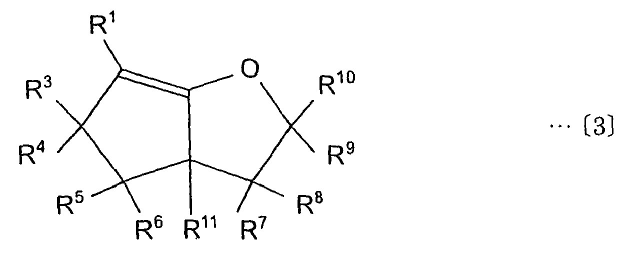
   wherein R¹ to R¹⁰ each independently represents hydrogen atom or an alkyl group having 1 to 20 carbon atoms, R¹¹ represents a condensed polycyclic hydrocarbon group or a group having at least three cyclic structures, and R¹² represents an alkyl group having 1 to 6 carbon atoms.
(2) A process for producing an optically active substance which comprises the steps of:
   bringing a 1-alkoxybicyclo [3.3.0] -2-oxaoctane compound represented by the formula [1], a 1-hydroxybicyclo [3.3.0] -2-oxaoctane compound represented by the formula [2] or a bicyclo [3.3.0] -2-oxa-1-octene compound represented by the formula [3], into reaction with a mixture of optically active substances having active hydrogen atom to form a mixture of diastereomers;
   resolving the mixture of the diastereomers into each diastereomer; and
   decomposing at least one of the diastereomers obtained by resolution to obtain an (R) optically active substance or an (S) optically active substance: 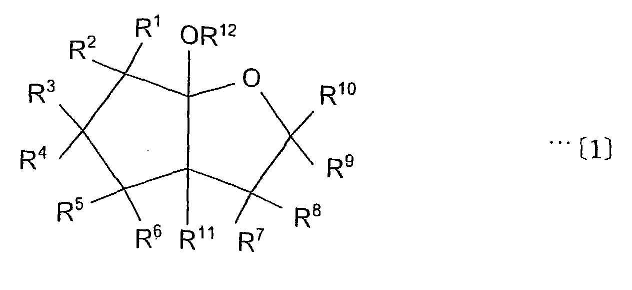 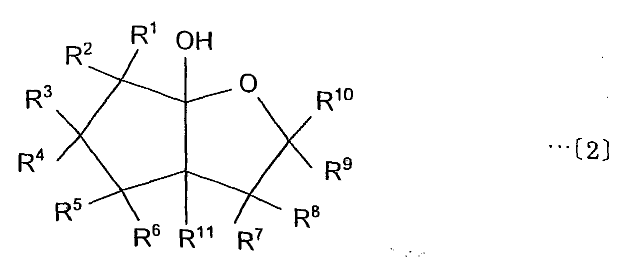 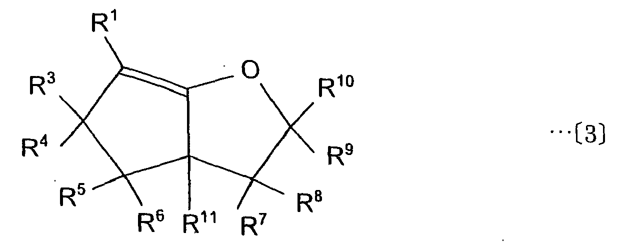
   wherein R¹ to R¹⁰ each independently represents hydrogen atom or an alkyl group having 1 to 20 carbon atoms, R¹¹ represents a condensed polycyclic hydrocarbon group or a group having at least three cyclic structures, and R¹² represents an alkyl group having 1 to 6 carbon atoms.
(3) A 1-alkoxybicyclo [3.3.0] -2-oxaoctane compound represented by the formula [1], a 1-hydroxybicyclo [3.3.0] -2-oxaoctane compound represented by the formula [2] or a bicyclo [3.3.0] -2-oxa-1-octene compound represented by the formula [3] : 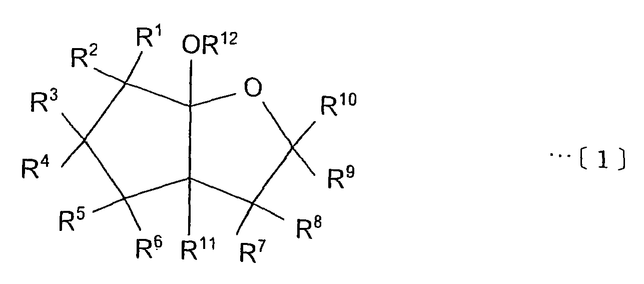 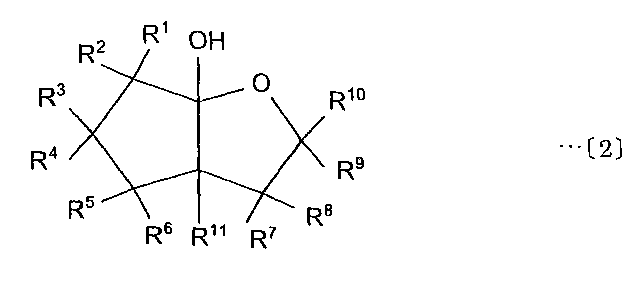 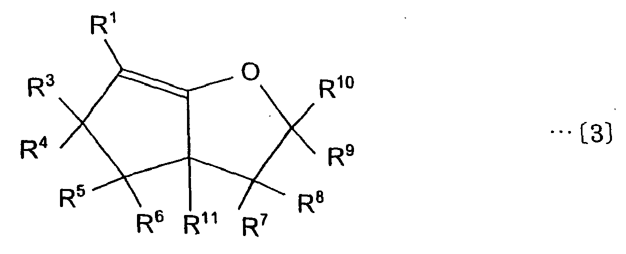 wherein R¹ to R¹⁰ each independently represents hydrogen atom or an alkyl group having 1 to 20 carbon atoms, R¹¹ represents fluorenylmethyl group or fluorenylidenemethyl group, and R¹² represents an alkyl group having 1 to 6 carbon atoms.
(4) A 1-methoxybicyclo [3.3.0] -2-oxaoctane compound represented by the formula [4] : 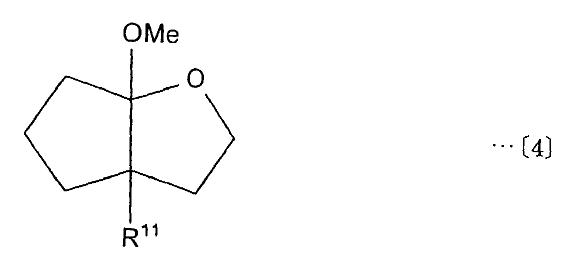
wherein R¹¹ represents bis(4-cyclohexylphenyl)methyl group, 4-(9-phenanthryl)phenyl group, 4-(1-pyrenyl)phenyl group, 4-(5-acenaphthenyl)phenyl group or 4-(9-anthryl)phenyl group.

### THE MOST PREFERRED EMBODIMENT TO CARRY OUT THE INVENTION

The agent for optical resolution of the present invention comprises a 1-alkoxybicyclo [3.3.0] -2-oxaoctane compound represented by the formula [1], a 1-hydroxybicyclo [3.3.0] -2-oxaoctane compound represented by the formula [2] or a bicyclo [3.3.0] -2-oxa-1-octene compound represented by the formula [3]. The process for producing an optically active substance of the present invention comprises bringing a 1-alkoxybicyclo [3.3.0] -2-oxaoctane compound represented by the formula [1], a 1-hydroxybicyclo [3.3.0] -2- oxaoctane compound represented by the formula [2] or a bicyclo [3.3.0] -2-oxa-1-octene compound represented by the formula [3] into reaction with a mixture of optically active substances having active hydrogen atom to form a mixture of diastereomers, resolving the mixture of diastereomers into each diastereomer, and decomposing at least one of the diastereomers obtained by the resolution to obtain an (R) optically active substance or an (S) optically active substance.

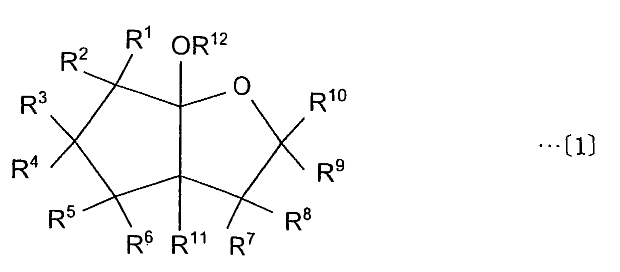

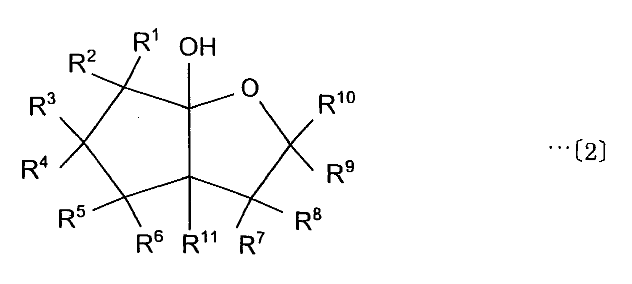

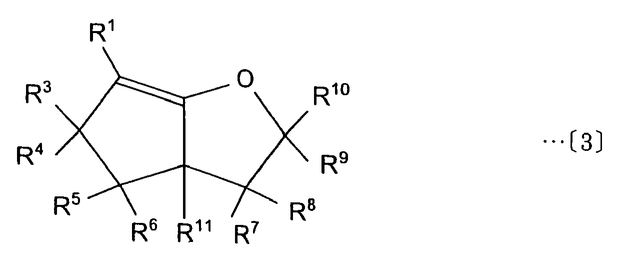

In formulae [1] to [3], R¹ to R¹⁰ each independently represent hydrogen atom or an alkyl group having 1 to 20 carbon atoms, R¹¹ represents a condensed polycyclic hydrocarbon group or a group having at least three cyclic structures, and R¹² represents an alkyl group having 1 to 6 carbon atoms.

Examples of the condensed polycyclic hydrocarbon group represented by R¹¹ include pentalenyl group, indenyl group, naphthyl group, tetrahydronaphthyl group, azulenyl group, heptalenyl group, biphenylenyl group, indacenyl group, acenaphthylenyl group, acenaphthenyl group, acenaphthenylphenyl group, fluorenyl group, fluorenylmethyl group, fluorenylphenyl group, fluorenylidenemethyl group, phenalenyl group, phenanthryl group, phenanthrylphenyl group, anthryl group, anthrylphenyl group, fluoranthenyl group, acephenanthrylenyl group, aceanthrylenyl group, triphenylenyl group, pyrenyl group, pyrenylphenyl group, chrysenyl group, naphthacenyl group, pleiadenyl group, pycenyl group, perylenyl group, pentaphenyl group, pentacenyl group, tetraphenylenyl group, hexaphenyl group, hexacenyl group, rubicenyl group, coronenyl group, trinaphthylenyl group, heptaphenyl group, heptacenyl group, pyranthrenyl group and ovalenyl group. Examples of the group having at least three cyclic structures represented by R¹¹ include triphenylmethyl group, tricyclohexylmethyl group, bis(diphenyl)methyl group and bis(cyclohexylphenyl)methyl group. Compounds having fluorenylmethyl group or fluorenylidenemethyl group among these groups are especially preferable due to the excellent ability of optical resolution.

The compound having fluorenylmethyl group or fluorenylidenemethyl group as the condensed polycyclic hydrocarbon group represented by R¹¹ can be produced, for example, in accordance with the reaction route shown in scheme [5] :

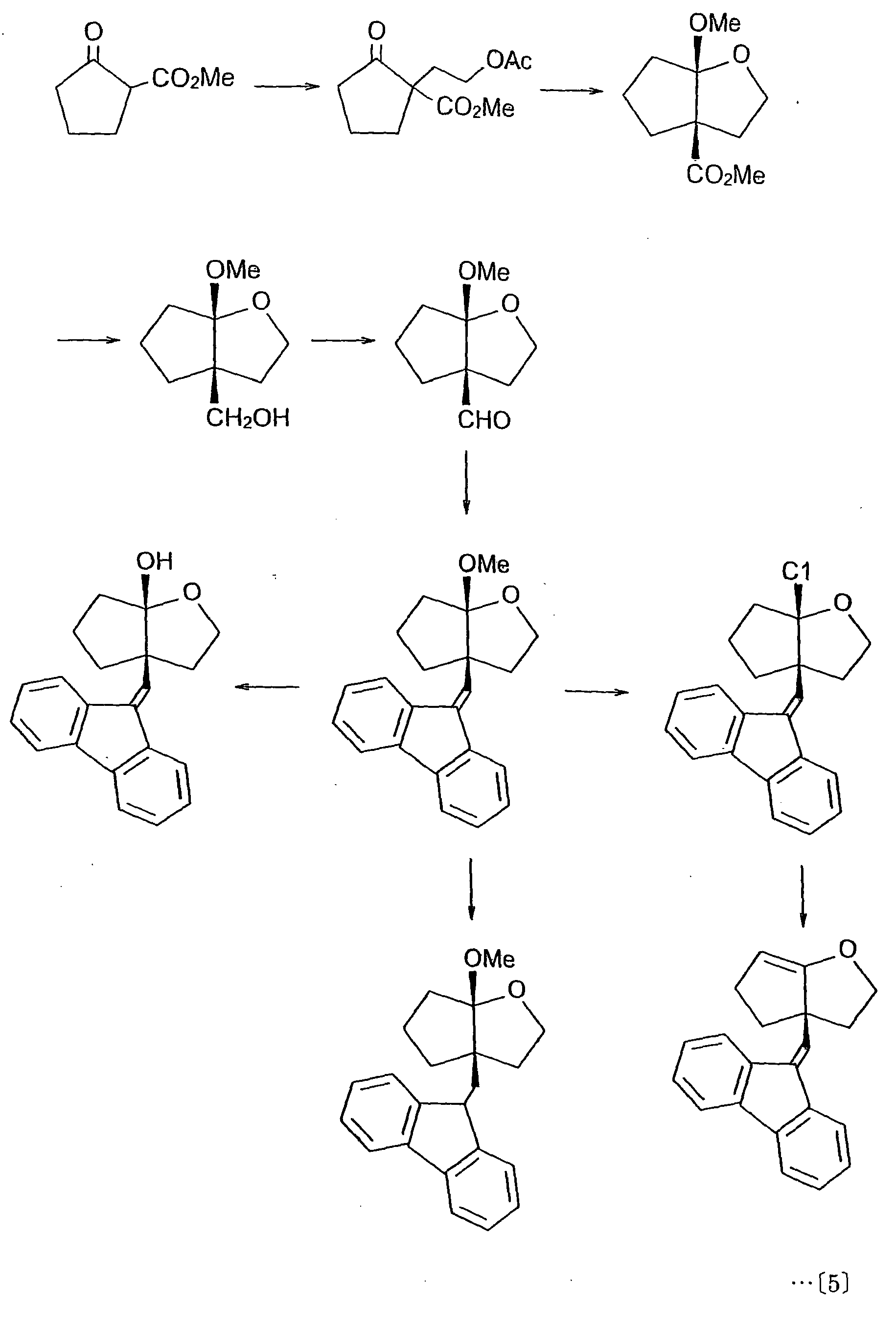

By the ring closure reaction of 2-(2-acetoxyethyl)-2-methoxy-carbonylcyclopentanone obtained by the reaction of 2-methoxycarbonyl-cyclopentanone and 2-bromoethyl acetate, 1-methoxy-5-methoxycarbonyl-bicyclo [3.3.0] -2-oxaoctane is obtained. Methoxycarbonyl group at the 5-position of 1-methoxy-5-methoxycarbonylbicyclo [3.3.0] -2-oxaoctane is reduced into hydroxymethyl group, which is then oxidized into formyl group, thereby obtaining 1-methoxy-5-formylbicyclo [3.3.0] -2-oxaoctane. By the dehydration reaction between 1-methoxy-5-formylbicyclo [3.3.0] -2-oxaoctane and fluorene, 1-methoxy-5-fluorenylidenemethylbicyclo- [3.3.0] -2-oxaoctane which is a compound represented by the formula [1] is obtained.

By hydrolysis of 1-methoxy-5-fluorenylidenemethylbicyclo [3.3.0] -2-oxaoctane, methoxy group at the 1-position is converted into hydroxyl group, thereby obtaining 1-hydroxy-5-fluorenylidenemethylbicyclo [3.3.0] -2-oxaoctane which is a compound represented by the formula [2] . By replacement of hydroxyl group at the 1-position with chlorine atom by the reaction of 1-methoxy-5-fluorenylidenemethylbicyclo [3.3.0] -2-oxaoctane with an acid chloride, followed by dehydrochlorination, 5-fluorenylidenemethylbicyclo [3.3.0] -2-oxa-1-octene which is a compound represented by the formula [3] can be obtained. By hydrogenation of the double bond in 1-methoxy-5-fluorenylidenemethylbicyclo [3.3.0] -2-oxaoctane in the presence of a catalyst, 1-methoxy-5-fluorenylmethylbicyclo [3.3.0] -2-oxaoctane can be obtained.

The compound having a condensed polycyclic hydrocarbon group as the group represented by R¹¹ can be produced, for example, in accordance with the reaction route shown in scheme [6] :

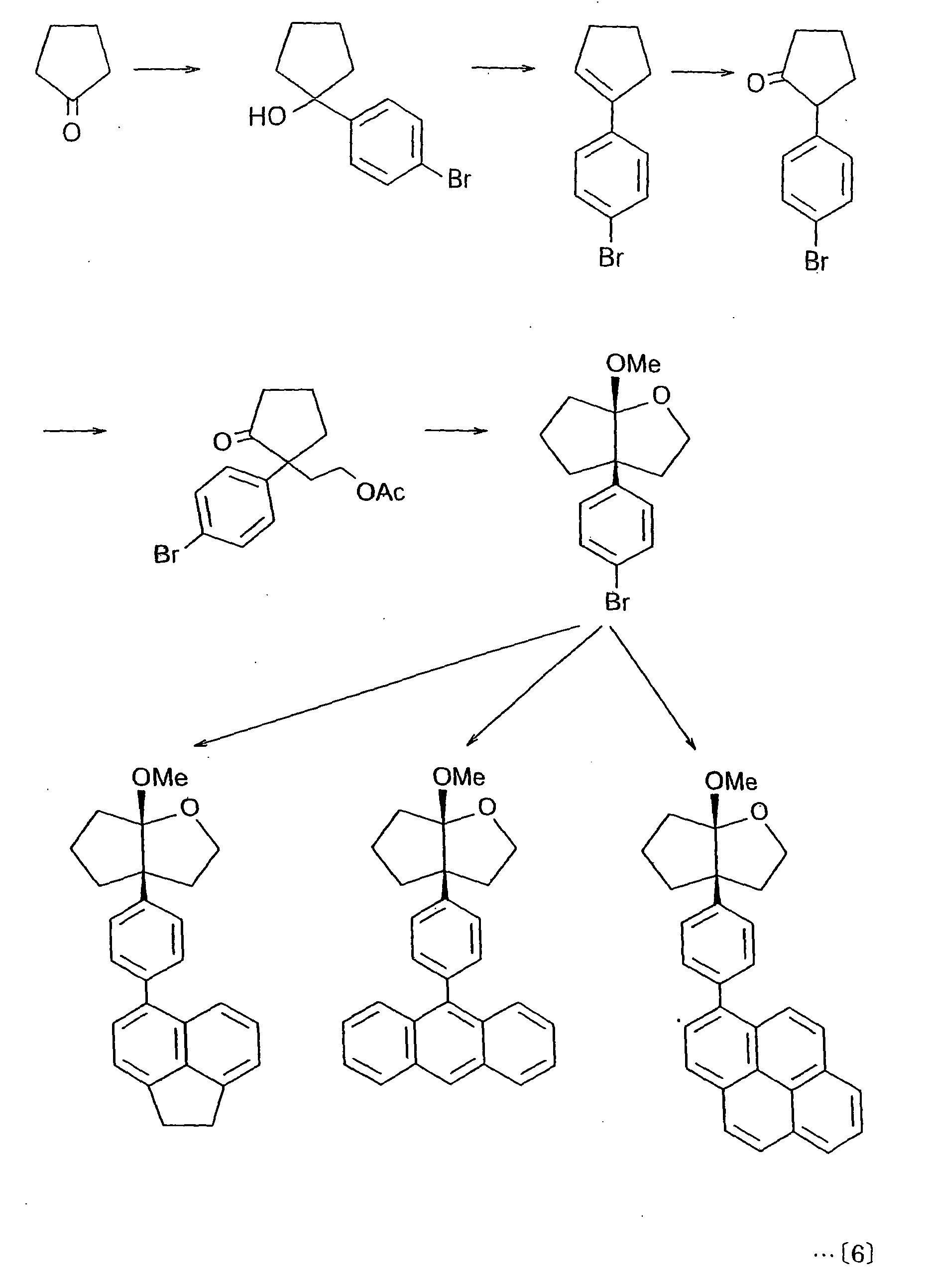

By dehydration of 1-(4-bromophenyl)cyclopentanol obtained by the reaction of cyclopentanone and 1,4-dibromobenzene, 4-bromo-1-cyclopenten-1-ylbenzene is obtained, and 2-(4-bromophenyl)cyclopentanone is obtained by further oxidation of 4-bromo-1-cyclopenten-1-ylbenzene. By the ring closure reaction of 2-(4-bromophenyl)-2-(2-acetoxyethyl)cyclopentanone obtained by the reaction of 2-(4-bromophenyl)cyclopentanone and 2-bromoethyl acetate, 1-methoxy-5-(4-bromophenyl)bicyclo [3.3.0] -2-oxaoctane is obtained. By replacing bromine atom with a condensed polycyclic hydrocarbon group by the reaction of 1-methoxy-5-(4-bromophenyl)bicyclo [3.3.0] -2-oxaoctane with an arylboronic acid having a condensed polycyclic hydrocarbon group, a derivative of 1-methoxy-5-phenylbicyclo [3.3.0] -2-oxaoctane having a condensed polycyclic hydrocarbon group can be obtained.

The compound having a group having at least three cyclic structures as the group represented by R¹¹ can be produced, for example, in accordance with the reaction route shown in scheme [7]:

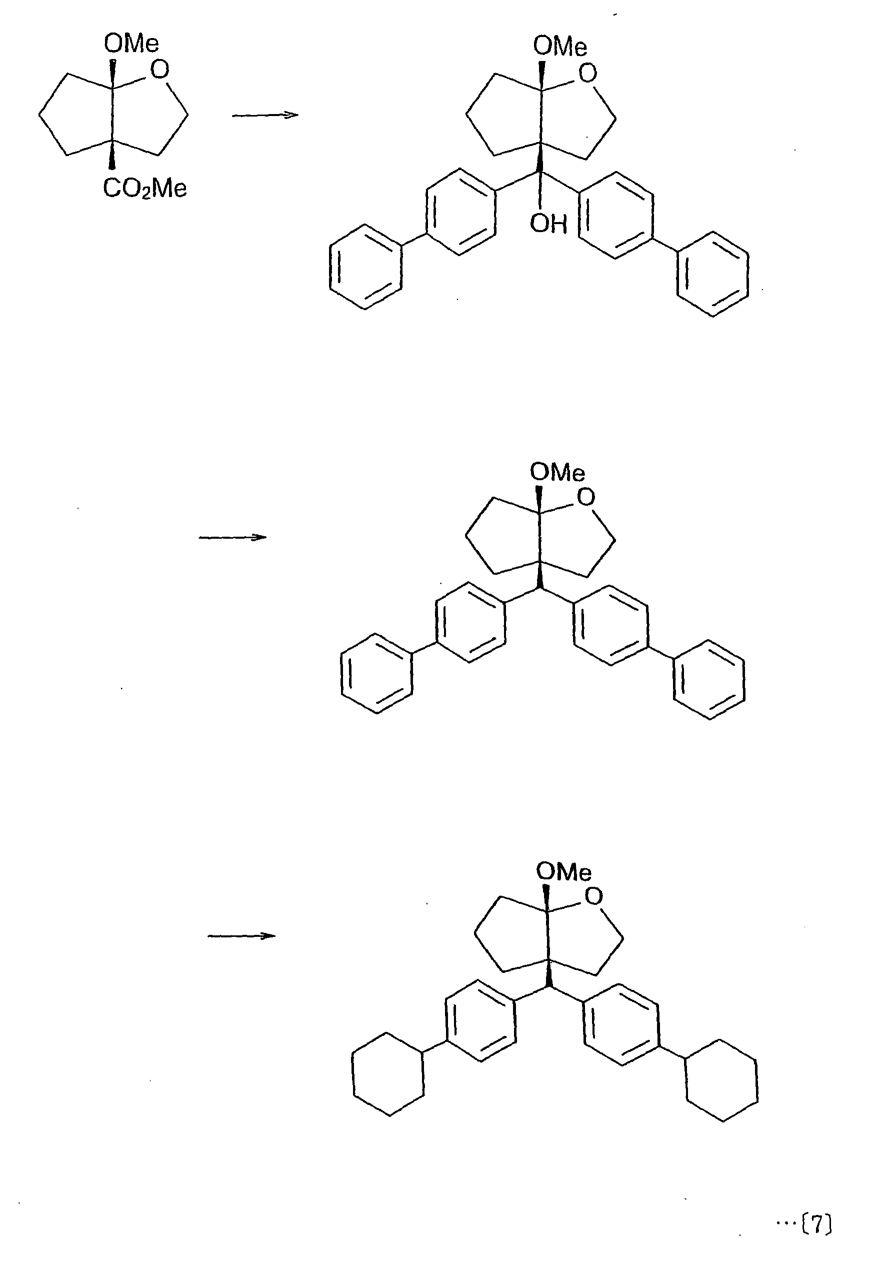

### Example 1

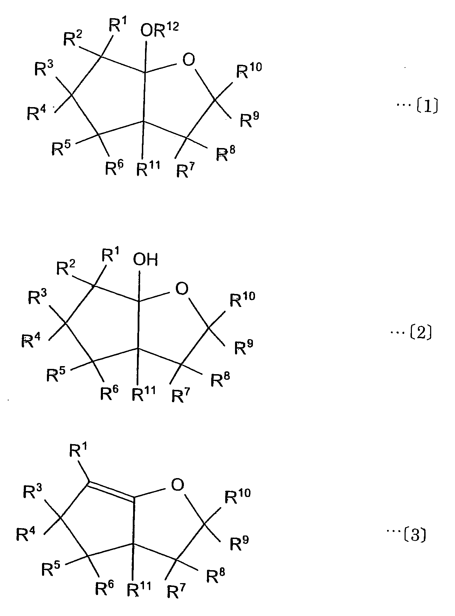

An ablation catheter equipped with a tip electrode having a shape shown in Figure 8 and a construction shown in Figure 9 was prepared. The tip electrode had a length of 8.0 mm, was made of platinum and had a shape such that three spherical surfaces having a diameter of 2.6 mm were arranged on the same straight line at a distance between the centers of 2.6 mm and the three spherical surfaces and the portion of the catheter shaft adjacent to the third spherical surface were connected to each other with a smooth surface. The catheter shaft had an effective length of 1,100 mm and an outer diameter of 2.4 mm, and the materials for the catheter shaft were a polyamide resin reinforced with braids of stainless steel for the portion at proximal end and a polyamide polyether elastomer for the bending portion at distal end. As the means for detecting the temperature of the tip electrode, an almel/chromel thermocouple was disposed at the inside of the tip electrode. Three electrodes made of platinum for measuring the electric potential were attached to the catheter shaft at a distance of 2 mm between each other from the tip electrode towards the portion at proximal end. A pair of wires running at the inside of the cavity of the tube from the portion for operation at proximal end to the distal end portion of the catheter shaft were disposed so that the distal end portion could be bent by pulling the wires by the operation of a handle at the portion for operation at proximal end. The tip electrode and the apparatus for generating radio frequency electric current were connected to each other through a lead wire for the electrode.

Figure 10 shows a diagram exhibiting the apparatus used for the cauterization test. An opposite electrode plate was placed at the bottom tetrazole salt, hydroxycarboxylic acid salts, hydroxyacid anhydrides, alcohols having a sulfoxide group, nitoso alcohols, alcohols having an azo group, hydroxyimines, hydroxyazides and aminoalcohols, which exhibit the optical activity. The optically active alcohol may have a linear structure or a cyclic structure.

Examples of the specific compound include 1-phenylethanol, 1-pbenyl-1,2-ethanediol, 1-phenyl-1,2-ethanediol 2-tosilate, 3-chloro-1,2-propanediol, 1-fluoro-3-pentyloxy-2-propanol, 1-fluoro-3-hexyloxy-2-propanol, 1-fluoro-3-heptyloxy-2-propanol, 1,2-propanediol 1-tosilate, propylene glycol, 1,3-butanediol, 2-amino-1-butanol, 1,4-dimethoxy-2,3-butanediol, 2-pentanol, 2,4-pentanediol, 2-methyl-2,4-pentanediol, 2-hexanol, 2-heptanol, 2,6-dimethyl-3,5-heptanediol, 1-fluoro-2-octanol, 2-nonanol, 1-fluoro-2-decanol, 2-hydroxy-1,2,2-triphenylethyl acetate, ethyl lactate, methyl 3-hydroxy-2-methylproplonate, 2-hydroxypropionamide, methyl butyrate, methyl 3-hydroxybutyrate, ethyl 3-hydroxybutyrate, t-butyl 3-hydroxybutyrate, methyl 4-bromo-3-hydroxybutyrate, sodium 3-hydroxybutyrate, pantotenyl alcohol, 2,2-dimethyl-1,3-dioxorane-4-methanol, menthol, borneol, 2-amino-2-phenylethanol, 2,3-glycerol carbonate tosilate, 2,3-glycerol carbonate 3-nitrobenzenesulfonate, hydrobenzoin, norephedrine, 1,1'-bi-2-naphthol and 4,4a,5,6,7,8-hexahydro-4a-methyl-2(3H)-naphthalenone.

Examples of the optically active amine include aminoalcohols, amines having an acetylene, amines having an olefin, aromatic amines, aminoaldehydes, aminoketones, amines having a protected amino group, amino acid esters, aminothioethers, aminothioesters, aminothioketones, nitro amines, nitrile amines, amino epoxy compounds, amines having an ether, amines having a sulfonic acid ester, halogenoamines, amines having an amide group, amines having a carbonate, amines having an oxime group, amines having an oxime ether group, isocyanatoamines, amines having a phosphoric acid ester, amines having a thiazole, amines having an oxazole, amines having an imidazole, amines having a triazole, amines having a tetrazole, amines having a protected imidazole, amines having a protected triazole, amines having a protected tetrazole, amines having a imidazole salt, amines having a triazole salt, amines having a tetrazole salt, amines having a sulfoxide group, amines having a thial group, nitroso amines, amines having an azo group, amines having an imino group, amines having an azide group, amines having a disulfide and amino acid salts, which exhibit the optical activity. The optically active amine may have a linear structure or a cyclic structure. Examples of the specific compound include 2-methylbutylamine, 1-cyclohexylethylamine, 1,2-diaminocyclohexane, cis-N-benzyl-2-(hydroxymethyl)cyclohexylamine and 1,2-diphenylethylenediamine.

Examples of the optically active carboxylic acid include alkylcarboxylic acid, acetylenecarboxylic acid, carboxylic acids having an olefin, aromatic carboxylic acids, carboxylic acids having an aldehyde, carboxylic acids having an ester, carboxylic acids having a disulfide, ketocarboxylic acids, amino acids, protected amino acids, carboxylic acids having a thioether, carboxylic acids having a thioester, carboxylic acids having a thioketone, carboxylic acids having nitro group, carboxylic acids having nitrile group, carboxylic acids having epoxy group, carboxylic acids having an ether, carboxylic acids having a sulfonic acid ester, halogenocarboxylic acids, carboxylic acids having an amide group, carboxylic acids having a carbonate, carboxylic acids having oxime group, carboxylic acids having oxime ether group, isocyanatocarboxylic acids, carboxylic acids having a phosphoric acid ester, carboxylic acids having a thiazole, carboxylic acids having an oxazole, carboxylic acids having an imidazole, carboxylic acids having a triazole, carboxylic acids having a tetrazole, carboxylic acids having a protected imidazole, carboxylic acids having a protected triazole, carboxylic acids having a protected tetrazole, carboxylic acids having an acid anhydride, carboxylic acids having an imidazole salt, carboxylic acids having a triazole salt, carboxylic acids having a tetrazole salt, carboxylic acid having a sulfoxide group, carboxylic acids having nitroso group, carboxylic acids having an azo group, carboxylic acids having an imino group, carboxylic acids having an azide group and amino acid salts, which exhibit the optical activity. The optically active carboxylic acid may have a linear structure or a cyclic structure. Examples of the specific compound include α-methoxyphenylacetic acid, 2-methoxy-2-(trifluoromethyl)phenylacetic acid, 2-phenylpropionic acid, {{(Phenylamino)carbonyl}oxy}propionic acid, epoxysuccinic acid, 2-amino- butyric acid, 2-phenylbutyric acid, 3-hydroxytetradecanoic acid, cis-2-benzamidocyclohexanecarboxylic acid, α-phenylglycine, p-hydroxy-phenylglycine, N-(3,5-dinitrobenzoyl)- α-phenylglycine and tetrahydro-2- furancarboxylic acid.

Examples of the optically active thiol include alkyl mercaptans, acetylene mercaptans, olefin mercaptans, aromatic mercaptans, mercaptoaldehydes, mercaptoketones, mercaptocarboxylic acids, aminomercaptans, protected aminomercaptans, mercaptothioethers, mrecaptothioesters, mercaptothioketones, nitro mercaptans, nitrile mercaptans, epoxy mercaptans, mercaptoethers, mercaptosulfonic acid esters, halogenomercaptans, amidomercaptans, mercaptocarbonates, mercaptans having an oxime group, mercaptans having an oxime ether group, mercaptocarboxylic acid esters, isocyanatomercaptans, mercaptophosphoric acid esters, mercaptans having a thiazole, mercaptans having an oxazole, mercaptans having an imidazole, mercaptans having a triazole, mercaptans having a tetrazole, mercaptans having a protected imidazole, mercaptans having a protected triazole, mercaptans having a protected tetrazole, mercaptans having an imidazole salt, mercaptans having a triazole salt, mercaptans having a tetrazole salt, mercaptocarboxylic acid salts, mercaptoacid anhydrides, mercaptans having a sulfoxide group, mercaptothials, nitrosomercaptans, mercaptans having an azo group, mercaptoimines, mercaptoazides and aminomercaptan salts, which exhibit the optical activity. The optically active thiol may have a linear structure or a cyclic structure. Examples of the specific compound include pentane-2-thiol.

The substituents at the 1-position and the 5-position in the 1-alkoxybicyclo [3.3.0] -2-oxaoctane compound represented by the formula [1] and the 1-hydroxybicyclo [3.3.0] -2-oxaoctane compound represented by the formula [2] have the cis-configuration. Compounds in which the substituents at the 1-position and the 5-position have the trans-configuration are almost entirely nonexistent. However, it is preferable that the agent for optical resolution of the present invention has a content of the cis-configuration of 99.9% by mole or greater and more preferably 99.999% by mole or greater. When addition takes place at the double bond in a bicyclo [3.3.0] -2-oxaoctene represented by the formula [3], wherby a substituent is introduced into the 1-position, the substituents at the 1-position and the 5-position have the cis-configuration, and almost no compounds in which the substituents at the 1-position and the 5-position have the trans-configuration are formed.

The function of the compounds represented by the formulae [1] to [3] of the present invention as the agent for optical resolution and the process for producing an optically active substance utilizing the compounds will be described in the following with reference to examples.

In the process of the present invention, when a 1-alkoxybicyclo-[3.3.0] -2-oxaoctane compound represented by the formula [1] and a racemic optically active alcohol represented by R¹³R¹⁴CHOH are brought into reaction with each other, the transetherification takes place at the 1-position as shown in scheme [8], and a mixture of two types of diastereomers is formed. The following scheme is shown with respect to a compound in which R¹ to R¹⁰ all represent hydrogen atom for simplicity.

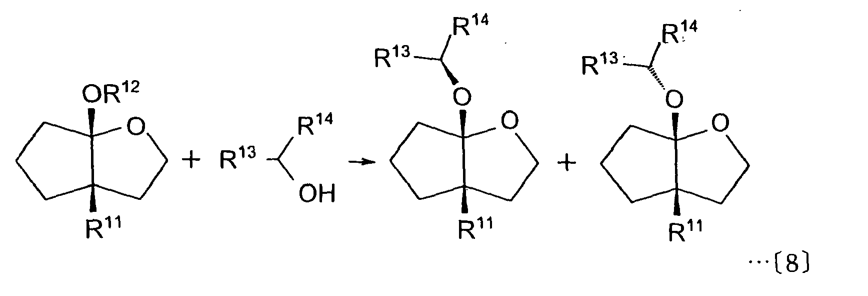

When a 1-alkoxybicyclo [3.3.0] -2-oxaoctane compound represented by the formula [1] and a racemic optically active amine represented by R¹⁵R¹⁶CHNH₂ are brought into reaction with each other, amination takes place at the 1-position in a similar manner, and a mixture of two types of diastereomers is formed. When a 1-alkoxybicyclo [3.3.0] -2-oxaoctane compound represented by the formula [1] and a racemic optically active carboxylic acid represented by R¹⁷R¹⁸CHCOOH are brought into reaction with each other, esterification takes place at the 1-position in a similar manner, and a mixture of two types of diastereomers is formed. When 1-alkoxybicyclo [3.3.0] -2-oxaoctane compound represented by the formula [1] and a racemic optically active thiol represented by R¹⁹R²⁰CHSH are brought into reaction with each other, the thioetherification takes place at the 1-position in a similar manner, and a mixture of two types of diastereomers is formed.

In the process of the present invention, when a 1-hydroxybicyclo-[3.3.0] -2-oxaoctane compound represented by the formula [2] and a racemic optically active alcohol, a racemic optically active amine, a racemic optically active carboxylic acid or a racemic optically active thiol are brought into reaction with each other, the etherification, the amination, the esterification or the thioetherification, respectively, takes place, and a corresponding mixture of two types of diastereomers is formed. When a bicyclo [3.3.0] -2-oxaoctene compound represented by the formula [3] and a racemic optically active alcohol, a racemic optically active amine, a racemic optically active carboxylic acid or a racemic optically active thiol are brought into reaction with each other, addition reaction to the double bond takes place, and a corresponding mixture of two types diastereomers is formed.

In the process of the present invention, a mixture of diastereomers having an optically active group at the 1-position of the bicyclo [3.3.0] -2-oxaoctane structure is resolved into each diastereomer. The process for the resolution is not particularly limited. Examples of the process include distillation, recrystallization, column chromatography and a process using a chromatographic resolution apparatus of the quasi-moving phase type. Since diastereomers have different chemical and physical properties, the diastereomers can be efficiently resolved by suitably selecting the process.

In the process of the present invention, at least one of the diastereomers obtained by the resolution is decomposed so that an (R) optically active substance or an (S) optically active substance is formed. The process for the decomposition of the diastereomer is not particularly limited. Examples of the process for decomposition include alcoholysis and hydrolysis in the presence of an acidic catalyst or a basic catalyst. By the alcoholysis of a diastereomer, an optically active substance having optically active hydrogen atom, such as an optically active alcohol, an optically active amine, an optically active carboxylic acid and an optically active thiol, having a very high optical purity and a 1-alkoxybicyclo [3.3.0] -2- oxaoctane compound represented by the formula [1] can be obtained. By the hydrolysis of a diastereomer, an optically active substance having optically active hydrogen atom, such as an optically active alcohol, an optically active amine, an optically active carboxylic acid and an optically active thiol, having a very high optical purity and a 1-hydroxybicyclo [3.3.0] -2- oxaoctane compound represented by the formula [2] can be obtained. The 1-alkoxybicyclo [3.3.0] -2-oxaoctane compound represented by the formula [1] and the 1-hydroxybicyclo [3.3.0] -2-oxaoctane compound represented by the formula [2] which are formed by the decomposition can be used repeatedly for the preparation of the mixture of diastereomers.

### EXAMPLES

The present invention will be described more specifically with reference to examples in the following. However, the present invention is not limited to the examples.

The measurements of the physical properties in the Examples were conducted in accordance with the following methods.

### (1) Melting point

The melting point was measured using an apparatus for measuring the melting point using a very small amount [manufactured by YANAGIMOTO SEISAKUSHO Co., Ltd.; MP-S3]. No correction was made.

### (2) Infrared absorption spectrum

The infrared absorption spectrum was measured using a Fourier transform infrared spectrophotometer [manufactured by NIPPON BUNKO Co., Ltd.; FT-IR/420] . The result is shown by the unit of cm⁻¹.

### (3) ¹H- and ¹³C-nuclear magnetic resonance spectra

The nuclear magnetic resonance spectra were measured using an apparatus for measuring the nuclear magnetic resonance [manufactured by NIPPON DENSHI Co., Ltd.; AL400] at 400 MHz (¹H) or 100 MHz (¹³C) at a temperature of 25°C using heavy chloroform as the solvent for the measurement. The chemical shift is shown as the δ-value using tetramethylsilane as the internal reference.

### (4) Mass analysis

The mass analysis was conducted using a high resolution mass analyzer [manufactured by NIPPON DENSHI Co., Ltd.; JMS-DX-303].

### (5) Elemental analysis

The elemental analysis was conducted using an elemental analyzer [manufactured by YANAGIMOTO SEISAKUSHO; CHN CODER MT-3] .

### (6) Liquid chromatography

The liquid chromatography was conducted using a high efficiency liquid chromatograph [manufactured by NIPPON WATERS Co., Ltd.; WATERS 600 System] and a differential refractometer [WATERS 2414].

### (7) Specific optical rotation

The specific optical rotation was measured using a digital polarimeter [manufactured by NIPPON BUNKO Co., Ltd.; DIP-370).

The following reagents were used for the reaction, and the reaction was always conducted under the atmosphere of argon.
(1) Methylene chloride: distilled over phosphorus pentaoxide
(2) Dry acetone: dried over potassium carbonate
(3) Dry pyridine: distilled over calcium hydride
(4) Dry tetrahydrofuran: purchased from KANTO KAGAKU Co., Ltd.

### Example 1

In accordance with the reaction route shown in scheme [9], (±)-1-methoxy-5-fluorenylidenemethylbicyclo [3.3.0] -2-oxaoctane and (±)-5-fluorenylidenemethylbicyclo [3.3.0] -2-oxa-1-octene were synthesized.

Utilizing the intermediate compound obtained in the reaction route shown by the scheme [9], (+)-1-methoxy-5-fluorenylidenemethylbicyclo [3.3.0] -2-oxaoctane was obtained.

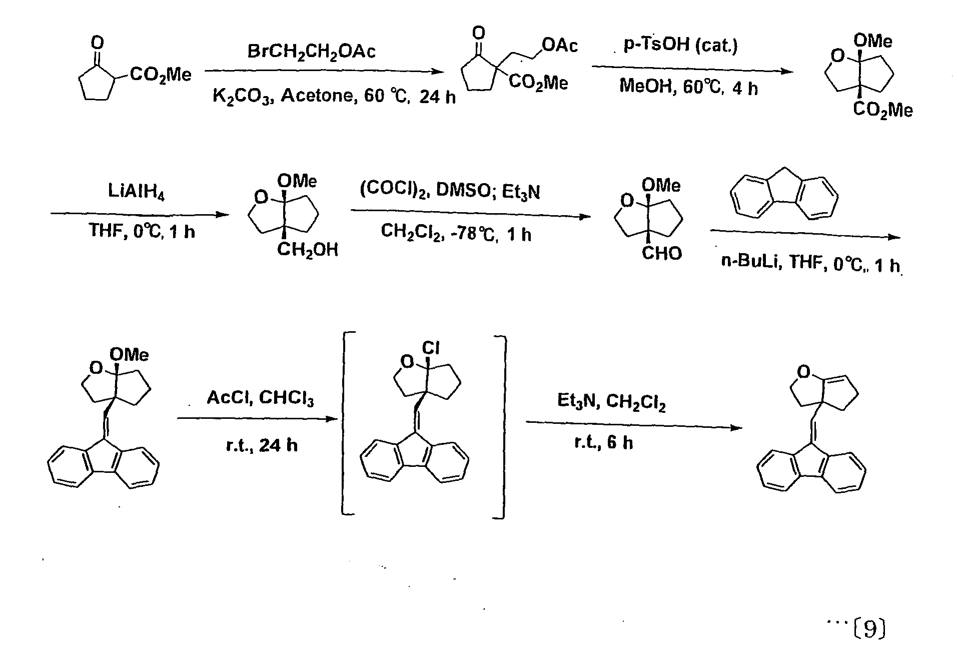

### (1) Synthesis of (±)-1-(2-acetoxyethyl)-2-oxocyclopentanecarboxylic acid methyl ester

To a dry acetone solution (500 ml) containing 31.0 g (0.218 moles) of 2-oxocyclopentanecarboxylic acid methyl ester and 40.0 g (0.240 moles) of 2-bromoethyl acetate, 45 g (0.326 moles) of potassium carbonate was added to prepare a suspension, and the prepared suspension was stirred at 60°C for 24 hours. After the suspension was cooled to the room temperature by leaving standing, the suspension was filtered. The filtrate was diluted with ethyl acetate (500 ml), washed with hydrochloric acid (0.1 mole/liter), water and a saturated aqueous solution of sodium chloride, dried over sodium sulfate and concentrated under a reduced pressure. The obtained residue was purified in accordance with the silica gel column chromatography using a hexane/ethyl acetate mixed solvent (the ratio of the amounts by volume: 3/1) as the elution solvent, and 34.8 g (0.152 moles) of (±)-1-(2-acetoxyethyl)-2-oxocyclopentanecarboxylic acid methyl ester was obtained as a colorless transparent liquid substance. The yield was 70%. The analytical data of the obtained compound are shown in the following.
FTIR (CHCl₃): 2958, 1735, 1435, 1232
¹H-NMR (400 MHz): 4.19 (dt, J=11.2, 6.8 Hz, 1H), one of -CH₂OAc, 4.08 (dt, J=11.2, 6.8 Hz, 1H), one of -CH₂OAc, 3.70 (s, 3H), -CO₂CH₃, 2.56 (ddd, J=12.6, 6.8, 2.0 Hz, 1H), one of -CH₂-C=O, 2.45 (dd, J=6.8, 4.8 Hz, 1H); 2.41 (d, J=8.4, 4.8 Hz, 1H), 2.31 (dd, J=14.4, 6.8 Hz, 1H), 2.25 (dd, J=14.4, 8.4 Hz, 1H), 2.00 (s, 3H).-OCOCH₃, 2.06-1.67 (m, 3H)
13C-NMR (100 MHz): 213.8 (C) carbon of ketone C=O, 170.8 (C) carbon of ester C=O, 170.6 (C) carbon of ester C=O, 60.9 (CH₂) methylene carbon adjacent to OAc, 58.5 (C) quaternary carbon, 52.7 (CH₃) methyl carbon of ester of CO₂Me, 37.5 (CH₂), 32.8 (CH₂), 32.5 (CH₂), 20.9 (CH₃) methyl carbon of acetate, 19.7 (CH₂)
HRMS (EI): Calcd. for C₁₁H₁₆O₅ (M⁺) 228.0998, Found: 228.0979

### (2) Synthesis of (±)-1-methoxy-5-methoxycarbonylbicyclo [3.3.0] -2-oxaoctane

To a methanol solution (100 ml) containing 22.8 g (0.1 mole) of (±)-1-(2-acetoxyethyl)-2-oxocyclopentanecarboxylic acid methyl ester, 86 mg (0.45 mmole) of p-toluenesulfonic acid monohydrate was added, and the resultant mixture was stirred at 60°C for 4 hours. Then, the solvent was removed by distillation under a reduced pressure. The obtained residue was purified in accordance with the silica gel column chromatography using a hexane/ethyl acetate mixed solvent (the ratio of the amounts by volume: 5/1) as the elution solvent, and 17.4 g (87 mmole) of (±)-1-methoxy-5-methoxycarbonylbicyclo [3.3.0] -2-oxaoctane was obtained as a colorless transparent liquid substance. The yield was 87%. The analytical data of the obtained compound are shown in the following.
FTIR (CHCl₃): 2952, 1734, 1435, 1274
¹H-NMR (400 MHz): 3.95 (dt, J=8.6, 4.4 Hz, 2H) CH₂ next to oxygen in the five-membered ring, 3.70 (s, 3H) methyl group of -CO₂Me, 3.26 (s, 3H) methyl group of -C-OMe, 2.75 (dt, J=12.8, 4.4 Hz, 1H), 2.45 (ddd, J=12.8, 10.4, 8.6 Hz, 1H), 2.08 (dd, J=10.4, 4.4 Hz, 1H), 1.82-1.80 (m, 1H); 1.76-1.56 (m, 4H)
¹³C-NMR (100 MHz): 174.0 (C) carbon of C=O of -CO₂Me, 120.3 (C) acetal quaternary carbon of -O-C-OMe, 67.9 (CH₂) methylene carbon adjacent to oxygen in the five-membered ring of -(CH₂)-O-, 62.3(C) quaternary carbon of -C-CO₂Me, 52.2 (CH₃) methyl carbon of ester of OMe, 51.3 (CH₃), 37.2 (CH₂), 35.8 (CH₂), 34.1 (CH₂), 22.7 (CH₂)
EI-HRMS (EI): Calcd. for C₁₀H₁₆O₄(M⁺): 200.1049, Found: 200.1050

### (3) Synthesis of (±)-1-methoxy-5-hydroxymethylbicyclo [3.3.0] -2-oxaoctane

To a tetrahydrofuran suspension (50 ml) containing 1.85 g (49 mmole) of lithium aluminum hydride cooled at 0°C, a tetrahydrofuran solution (50 ml) containing 10.0 g (50 mmole) of (±)-1-methoxy-5-methoxycarbonylbicyclo [3.3.0] -2-oxaoctane was added dropwise, and the resultant mixture was stirred at 0°C for 1 hour. Then, the reaction was terminated by adding 2 ml of a tetrahydrofuran/water mixture (the ratio of amounts by volume: 1/1) in small portions, and 200 ml of ether and 200 ml of water were added. The obtained aqueous layer was treated by extraction 3 times each with 150 ml of ether. The organic layers were combined, washed with a saturated aqueous solution of sodium chloride, dried over magnesium sulfate and concentrated under a reduced pressure. The obtained residue was purified in accordance with the silica gel column chromatography using a hexane/ethyl acetate mixed solvent (the ratio of the amounts by volume: 3/1) as the elution solvent, and 7.74 g (45 mmole) of (±)-1-methoxy-5-hydroxymethylbicyclo [3.3.0] -2-oxaoctane was obtained as a colorless transparent liquid substance. The yield was 90%. The analytical data of the obtained compound are shown in the following.
FTIR (CHCl₃): 3536, 2926, 1510, 1449
¹H-NMR (400 MHz): 3.87 (dt, J=8.0, 2.0 Hz, 1H) one of CH₂ adjacent to oxygen in the ring, 3.74 (ddd, J=10.2, 8.0, 6.4 Hz, 1H) one of CH₂ adjacent to oxygen in the ring, 3.55 (s, 2H) methylene hydrogen of CH₂OH in the side chain, 3.23 (s, 3H) MeO-, 3.02 (br s, 1H) OH, 2.11 (dd, J=6.0, 2.4 Hz, 1H), 2.08 (dd, J=6.0, 2.4 Hz, 1H), 1.72 (ddd, J=11.2, 10.2, 8.0 Hz, 1H), 1.67 (dd, J=8.0, 3.6 Hz, 1H), 1.64 (dd, J=3.6, 2.4 Hz, 1H), 1.58 (ddd, J=11.2, 8.0, 6.0 Hz, 1H), 1.54-1.45 (2H, m)
¹³C-NMR (100 MHz): 119.3 (C), 66.9 (CH₂), 66.4 (CH₂), 55.8 (C), 50.7 (CH₃), 37.4 (CH₂), 35.1 (CH₂), 35.0 (CH₂), 21.8 (CH₂)
HRMS (EI): Calcd. for C₉H₁₆O₃: 172.1099, Found: 172.1096

### (4) Synthesis of (±)-1-methoxy-5-formylbicyclo [3.3.0] -2-oxaoctane

To a dry methylene chloride solution (200 ml) containing 3.87 g (30.5 mmole) of oxalyl dichloride, a dry methylene chloride solution (20 ml) containing 2.72 g (34.9 mmole) of dimethyl sulfoxide was added dropwise at -78°C over 5 minutes. To the solution of the reaction mixture, a dry methylene chloride solution (50 ml) containing 5.0 g (29 mmole) of (±)-1-methoxy-5-hydroxymethylbicyclo [3.3.0] -2-oxaoctane was slowly added dropwise at -78°C. After the obtained mixture was stirred at -78°C for 1 hour, 8.8 g (87 mmole) of triethylamine was added. The temperature was slowly elevated, and the stirring was continued at the room temperature for 1 hour. To the obtained mixture, 100 ml of water was added, and the resultant mixture was treated by extraction 3 times each with 150 ml of ether. The organic layers were combined, washed with 50 ml of a hydrochloric acid (0.1 mole/liter), a saturated aqueous solution of sodium hydrogencarbonate, water and a saturated aqueous solution of sodium chloride, dried over sodium sulfate and concentrated under a reduced pressure. The obtained residue was purified in accordance with the silica gel column chromatography using a hexane/ethyl acetate mixed solvent (the ratio of the amounts by volume: 3/1) as the elution solvent, and 4.7 g (27.6 mmole) of (±)-1-methoxy-5-formylbicyclo [3.3.0] -2-oxaoctane was obtained as a colorless transparent liquid substance. The yield was 95%. The analytical data of the obtained compound are shown in the following.
FTIR (CHCl₃): 2955, 1768, 1452
¹H-NMR (400 MHz): 9.44 (s, 1H), 3.99 (td, J=8.4, 2.4 Hz, 1H), 3.86 (ddd, J=10.4, 8.4, 6.4 Hz, 1H), 3.26 (s, 3H), 2.66 (ddd, J=12.8, 6.0, 2.4 Hz, 1H), 2.24 (ddd, J=12.8, 11.2, 6.8 Hz, 1H), 2.14 (ddd, J=11.2, 6.0, 1.6 Hz, 1H), 1.84 (dd, J=12.0, 6.8 Hz, 1H), 1.75 (td, J=12.0, 6.0 Hz, 1H), 1.67 (dd, J=12.8, 2.0 Hz, 1H), 1.67 (dd, J=8.2, 2.0 Hz, 1H), 1.46 (ddd, J=12.8, 6.0, 1.6 Hz, 1H)
13C-NMR (100 MHz): 200.1 (C), 121.4 (C), 67.7 (CH₂), 65.2 (C), 51.3 (CH₃), 34.9 (CH₂), 32.5 (CH₂), 23.3 (CH₂)
HRMS (EI): Calcd. for C₉H₁₄O₃: 170.0943, Found: 170.0938

### (5) Synthesis of (+)-1-methoxy-5-formylbicyclo [3.3.0] -2-oxaoctane

Into a flask attached with a stirrer and a refluxing condenser, a dry toluene solution (350 ml) containing 300 g of (±)-1-methoxy-5-(2-propenyl)bicyclo [3.3.0] -2-oxaoctane was placed. Molecular sieves 5A in an amount of 300 g and 228 g of {(1S)-endo}-(-)-borneol was added, and 6,000 g of molecular sieves 4A was packed into the refluxing condenser. The obtained mixture was heated at 110°C for 10 hours under stirring, and the formed methanol was adsorbed with molecular sieves 4A. The reaction fluid was filtered, and the filtrate was concentrated under a reduced pressure to obtain 520 g of a residue. The obtained residue was resolved and purified in accordance with the silica gel column chromatography using a hexane/ether mixed solvent (the ratio of the amounts by volume: 40/1) as the elution solvent, and 171 g of a diastereomer having a greater Rf value ([α]_{d}²⁵=-74.2, c1.05, CHCl₃) and 189 g of a diastereomer having a smaller Rf value ([α]_{d}²⁵=+5.6, c0.84, CHCl₃) were obtained.

To a methylene chloride solution (200 ml) of the diastereomer having a greater Rf value, 16.2 g of methanol and 14.0 g of pyridinium p-toluenesulfonate was added, and the resultant mixture was stirred at the room temperature for 30 minutes. The reaction mixture was washed with a saturated aqueous solution of sodium chloride, dried with anhydrous potassium carbonate and filtered. The residue obtained by concentrating the filtrate under a reduced pressure was purified in accordance with the silica gel column chromatography using a hexane/ethyl acetate mixed solvent (the ratio of the amounts by volume: 19/1) as the elution solvent, and 92 g of (+)-1-methoxy-5-(2-propenyl)-bicyclo [3.3.0] -2-oxaoctane was obtained.

To a benzene solution (800 ml) containing 80 g (0.439 mole, [α] _{d}²⁵=+54.8, c0.80, CHCl₃) of (+)-1-methoxy-5-(2-propenyl)bicyclo [3.3.0] - 2-oxaoctane, 11.8 g (30.8 mmole) of dichlorobis(benzonitrile)- palladium(II) was added at the room temperature, and the resultant mixture was stirred for 30 minutes. Then, 9 ml of triethylamine was added, and the obtained mixture was stirred at the room temperature for 10 minutes. The reaction fluid was diluted with ether and filtered through celite. The residue obtained by concentration of the filtrate was purified in accordance with the silica gel column chromatography using a hexane/ethyl acetate mixed solvent (the ratio of the amounts by volume: 20/1) as the elution solvent, and 64.2 g (0.353 moles) of (+)-1-methoxy-5-(1-propenyl)bicyclo [3.3.0] -2-oxaoctane was obtained as a colorless oily liquid substance. The yield was 80%.

Through a methylene chloride solution (275 ml) containing 55 g (0.302 moles) of (+)-1-methoxy-5-(1-propenyl)bicyclo [3.3.0] -2-oxaoctane obtained above, a stream of ozone-oxygen gas was passed at -78°C for 16 hours. When the ozonization was completed, ozone in an excess amount was removed by passing dry nitrogen gas through the reaction fluid at -78°C for 30 minutes. Then, 38.3 ml of methyl sulfide was added, and the resultant mixture was stirred until the temperature was elevated to the room temperature. The reaction fluid was poured into water, and the product was obtained by extraction with methylene chloride. The organic layer was washed with a saturated aqueous solution of sodium chloride, dried with anhydrous potassium carbonate and filtered. Byproducts at the side of greater polarity in the residue obtained by concentration of the filtrate under a reduced pressure were removed with a short column packed with silica gel using a hexane/ethyl acetate mixed solvent (the ratio of the amounts by volume: 8/1) as the elution solvent, and (+)-1-methoxy-5-formylbicyclo [3.3.0] -2-oxaoctane ([α]_{d}²⁵=+90.5, c2.02, CHCl₃) was obtained as a colorless oily liquid substance.

### (6) Synthesis of (±)-1-methoxy-5-fluorenylidenemethylbicyclo [3.3.0] -2-oxaoctane

To a dry tetrahydrofuran solution (50 ml) containing 3.9 g (23.5 mmole) of fluorene, 10.6 ml (25.9 mmole) of a 2.44 mole/liter hexane solution of butyllithium was added dropwise at 0°C, and the resultant mixture was stirred at 0°C for 30 minutes. Then, a dry tetrahydrofuran solution (30 ml) containing 4.0 g (23.5 mmole) of (±)-1-methoxy-5-formylbicyclo [3.3.0] -2-oxaoctane was added. After the reaction mixture was stirred at 60°C for 6 hours, water was added. The obtained reaction mixture was treated by extraction 3 times each with 150 ml of ether. The organic layers were combined, washed with 50 ml of a hydrochloric acid (0.1 mole/liter), a saturated aqueous solution of sodium hydrogencarbonate, water and a saturated aqueous solution of sodium chloride, dried over sodium sulfate and concentrated under a reduced pressure. The obtained residue was purified in accordance with the silica gel column chromatography using a hexane/ethyl acetate mixed solvent (the ratio of the amounts by volume: 5/1) as the elution solvent, and 6.50 g (20.5 mmole) of (±)-1-methoxy-5-fluorenylidenemethylbicyclo[3.3.0] -2-oxaoctane was obtained as a colorless liquid substance. The yield was 87%. The analytical data of the obtained compound are shown in the following.
FTIR (CHCl₃): 3055, 2960, 2830, 1634, 1447
¹H-NMR (400 MHz): 7.92 (d, J=7.6 Hz, 1H), 7.80 (d, J=6.4 Hz, 1H), 7.78 (d, J=7.6 Hz, 1H), 7.72 (d, J=6.4 Hz, 1H), 7.60 (s, 1H), 7.40 (t, J=6.4 Hz, 2H), 7.34 (t, J=7.6 Hz, 2H), 4.07 (td, J=8.0, 4.8 Hz, 1H), 3.93 (dt, J=8.0, 8.0 Hz, 1H), 3.51 (s, 2H), 2.64 (dt, J=12.0, 7.6 Hz, 1H), 2.48 (ddd, J=12.0, 7.6, 4.8 Hz, 1H), 2.45-2.23 (m, 3H), 1.85-1.72 (m. 3H)
¹³C-NMR (100 MHz): 141.4 (C), 140.4 (C), 138.0 (C), 135.3 (C), 135.3 (C), 134.1 (CH), 127.7 (CH), 127.3 (CH), 126.8 (CH), 126.3 (CH), 125.5 (CH), 119.8 (CH), 119.7 (CH), 119.2 (CH), 118.5 (C), 66.8 (CH₂), 55.4 (C), 51.1 (CH₃), 38.9 (CH₂), 38.4 (CH₂), 32.8 (CH₂), 22.3 (CH₂)
HRMS (EI): Calcd. for C₂₂H₂₂O₂: 318.1620, Found: 318.1619

### (7) Synthesis of (+)-1-methoxy-5-fluorenylidenemethylbicyclo [3.3.0] -2-oxaoctane

In accordance with the same procedures as those conducted above except that (+)-1-methoxy-5-formylbicyclo [3.3.0] -2-oxaoctane ([α]_{d}²⁵= +90.5, c2.02, CHCl₃) was used as the starting material, (+)-1-methoxy-5-fluorenylidenemethylbicyclo [3.3.0] -2-oxaoctane ([α]_{d}²⁵=+42.1, c0.918. CHCl₃) was synthesized.

### (8) Synthesis of (±)-5- fluorenylidenemethylbicyclo [3.3.0] -2-oxa-1-octene

To a chloroform solution (50 ml) containing 2.0 g (6.29 mmole) of (±)-1-methoxy-5-fluorenylidenemethylbicyclo [3.3.0] -2-oxaoctane, 4.47 ml (62.9 mmole) of acetyl chloride was added, and the resultant mixture was stirred at the room temperature for 24 hours. While the care was taken to prevent contamination with moisture, the obtained reaction mixture was concentrated under a reduced pressure, and chloroform, acetyl chloride in an excess amount and methyl acetate formed by the reaction were removed by distillation. After the obtained residue was diluted with 30 ml of methylene chloride, the diluted solution was added dropwise to a methylene chloride solution (30 ml) containing 8.7 ml (62.9 mmole) of triethylamine, and the reaction mixture was stirred at the room temperature for 2 hours. The obtained reaction mixture was poured into 100 ml (0.5 moles) of a 5 mole/liter aqueous solution of sodium hydroxide under vigorous stirring, and then the obtained mixture was treated by extraction 3 times each with 150 ml of ether. The organic layers were combined, washed with water, dried over potassium carbonate and concentrated under a reduced pressure. The obtained solid residue was recrystallized using a hexane/ethyl acetate mixed solvent (the ratio of the amounts: 5/1), and 1.51 g (5.28 mmole) of (±)- 5-fluorenylidenemethylbicyclo [3.3.0] -2-oxa-1-octene was obtained. The yield was 84%. The analytical data of the obtained compound are shown in the following.
FTIR (CHCl₃): 3055, 2971, 2850, 1604, 1446
¹H-NMR (400 MHz): 7.64 (d, J=6.8 Hz, 2H), 7.58 (d, J=7.4 Hz, 2H), 7.29 (t, J=7.4 Hz, 1H), 7.23 (t, J=6.2 Hz, 2H), 7.18 (t, J=7.4, 1H), 6.99 (s, 1H), 4.50 (d, J=2.4 Hz, 1H), 4.46 (t, J=8.6 Hz, 1H), 4.32 (ddd, J=11.6, 8.6, 5.2 Hz, 1H), 2.63 (ddd, J=14.2, 8.8, 5.2 Hz, 1H), 2.47 (dd, J=8.8, 5.2 Hz, 1H), 2.44 (dd, J=8.8, 6.0 Hz, 1H), 2.36 (ddd, J=14.2, 8.0, 2.8 Hz, 1H), 2.14 (td, J=11.6, 2.8 Hz, 1H), 2.11 (td, J=8.8, 5.2 Hz, 1H)
¹³C-NMR (100 MHz): 168.2 (C), 140.9 (C), 138.7 (C), 138.2 (C), 137.0 (C), 134.6 (C), 134.1 (CH), 127.8 (CH), 127.5 (CH), 126.7 (CH), 126.3 (CH), 125.6 (CH), 119.7 (CH), 119.5 (CH), 119.1 (CH), 89.2 (CH), 76.4 (CH₂), 55.4 (C), 38.3 (CH₂), 37.8 (CH₂), 33.4 (CH₂)
HRMS (EI): Calcd. for C₂₁H₁₈O: 286.1358, Found: 286.1338

### Example 2

In accordance with the reaction route shown in scheme [10], (±)-1-methoxy-5-isopropenylbicyclo [3.3.0] -2-oxaoctane was synthesized.

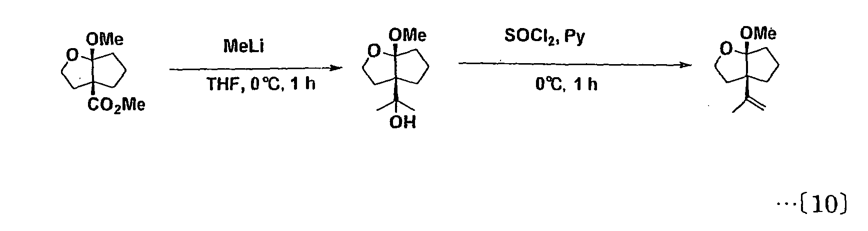

To a dry tetrahydrofuran solution (50 ml) containing 3.0 g (15.0 mmole) of (±)-1-methoxy-5-methoxycarbonylbicyclo [3.3.0] -2-oxaoctane, 3.16 ml (3.6 mmole) of a 1.14 moles/liter hexane solution of methyllithium was added at 0°C, and the resultant mixture was stirred for 1 hour. Then, water was added, and the obtained solution was treated by extraction 3 times each with 200 ml of ether. The organic layers were combined, washed with a saturated aqueous solution of sodium chloride, dried over sodium sulfate and concentrated under a reduced pressure. The obtained crude (±)-1-methoxy-5-dimethylhydroxymethylbicyclo-[3.3.0] -2-oxaoctane was used for the reaction in the next step without purification.

To a dry pyridine solution (6 ml) of (±)-1-methoxy-5-dimethyl-hydroxymethylbicyclo [3.3.0] -2-oxaoctane, 2.0 ml (27.4 mmole) of thionyl chloride was added, and the resultant mixture was stirred at the room temperature for 1 hour. Then, water was added, and the obtained solution was treated by extraction 3 times each with 100 ml of ether. The organic layers were combined, washed with a saturated aqueous solution of sodium chloride, dried over sodium sulfate and concentrated under a reduced pressure. The obtained residue was purified in accordance with the silica gel column chromatography using a hexane/ethyl acetate mixed solvent (the ratio of the amounts by volume: 10/1) as the elution solvent, and 0.96 g (5.25 mmole) of (±)-1-methoxy-5-isopropenylbicyclo [3.3.0] -2-oxaoctane was obtained as a colorless liquid substance. The yield was 35%. The analytical data of the obtained compound are shown in the following.
FTIR (CHCl₃): 3055, 2960, 2830, 1634, 1447
¹H-NMR (400 MHz): 4.84 (s, 2H), 3.98 (td, J=8.0, 4.8 Hz, 1H), 3.91 (td, J=8.0, 8.0 Hz, 1H), 3.30 (s, 3H), 2.35 (dt, J=12.4, 8.0 Hz, 1H), 2.13 (ddd, J=10.8, 7.2, 1.6 Hz, 1H), 1.94 (dt, J=12.4, 7.2 Hz, 1H), 1.90 (dt, J=7.2, 4.8 Hz, 1H), 1.86 (s, 3H), 1.82-1.69 (m, 3H), 1.65-1.60 (m, 1H)
13C-NMR (100 MHz): 147.3 (C), 118.1 (C), 110.8 (CH₂), 66.4 (CH₂), 59.3 (C), 51.0 (CH₃), 36.8 (CH₂), 36.7 (CH₂), 34.4 (CH₂), 21.6 (CH₃), 21.4 (CH₂)
HRMS (EI): Calcd. for C₁₁H₁₈O₂: 182.1307, Found: 182.1309

### Example 3

In accordance with the reaction route shown in scheme [11], (±)-1-methoxy-5-bis(4-cyclohexylphenyl)methylbicyclo [3.3.0] -2-oxaoctane was synthesized.

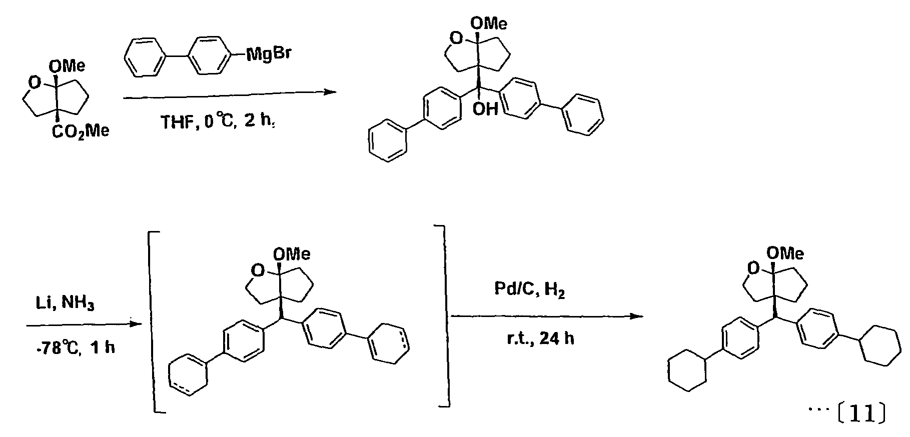

### (1) Synthesis of (±)-1-methoxy-5-bis(4-biphenyl)hydroxymethylbicyclo-[3.3.0] -2-oxaoctane

In 200 ml of dry tetrahydrofuran, a dry tetrahydrofuran solution (50 ml) containing 3.0 g (15 mmole) of (±)-1-methoxy-5-methoxycarbonylbicyclo [3.3.0] -2-oxaoctane was added dropwise to 4-biphenyl-magnesium bromide prepared from 1.26 g (52.5 mmole) of magnesium foil and 10.5 g (45.0 mmole) of 4-bromobiphenyl at 0°C, and the resultant mixture was stirred at 60°C for 2 hours. Then, an aqueous solution of ammonium chloride was poured into the reaction mixture, and the obtained mixture was treated by extraction 3 times each with 100 ml of ether. The organic layers were combined, washed with a saturated aqueous solution of sodium chloride, dried over sodium sulfate and concentrated under a reduced pressure. The obtained residue was purified in accordance with the silica gel column chromatography using a hexane/ethyl acetate mixed solvent (the ratio of the amounts by volume: 10/1) as the elution solvent, and 6.07 g (12.75 mmole) of (±)-1-methoxy-5-bis(4-biphenyl)hydroxymethylbicyclo [3.3.0] -2-oxaoctane was obtained as colorless crystals. The yield was 85%. The analytical data of the obtained compound are shown in the following.
FTIR (CHCl₃): 3355, 2960, 2830, 1634, 1447
¹H-NMR (400 MHz): 7.64 (t, J=8.8 Hz, 4H), 7.59 (d, J=8.8 Hz, 4H), 7.54 (s, 4H), 7.46 (t, J=10.0 Hz, 4H), 7.36 (t, J=10.0 Hz, 2H), 6.32 (s, 1H), 4.05 (td, J=10.8, 5.6 Hz, 1H), 3.91 (dt, J=10.8, 10.0 Hz, 1H), 3.44 (s, 3H), 2.97 (ddd, J=16.0, 10.0, 5.6 Hz, 1H), 2.80 (dt, J=16.4, 11.2 Hz, 1H), 2.24 (dt, J=16.4, 10.8 Hz, 1H), 2.02 (dd, J=14.4, 11.2 Hz, 1H), 1.96 (dd, J=16.4, 12.4 Hz, 1H), 1.62 (dd, J=14.4, 10.8 Hz, 1H), 1.35-1.28 (m, 1H), 1.10 (dt, J=14.4, 13.2 Hz), 1H)
¹³C-NMR (100 MHz): 146.3 (C), 145.6 (C), 140.7 (C), 140.5 (C), 139.1 (C), 138.9 (C), 129.1 (CHx2), 128.7 (CHx3), 128.7 (CHx3), 127.2 (CHx2), 127.0 (CHx2), 126.9 (CHx2), 126.1 (CHx2), 125.5 (CHx2), 120.9 (C), 82.6 (C), 66.5 (CH₂), 61.9 (C), 50.5 (CH₃), 38.4 (CH₂), 37.2 (CH₂), 32.6 (CH₂), 20.9 (CH₂)
HRMS (EI): Calcd. for C₃₃H₃₂O₃: 476.2351, Found: 476.2354

### (2) Synthesis of (±)-1-methoxy-5-bis(4-cyclohexylphenyl)methylbicyclo-[3.3.0] -2-oxaoctane

To a dry tetrahydrofuran solution (10 ml) containing 2.38 g (5.0 mmole) of (±)-1-methoxy-5-bis(4-biphenyl)hydroxymethylbicyclo [3.3.0] - 2-oxaoctane, 30 ml of liquid ammonia was added. To the obtained solution, 175.0 mg (25.0 mmole) of lithium was added at -78°C. After the obtained deep blue solution was stirred at -78°C for 1 hour, a saturated aqueous solution of ammonium chloride was added, and the resultant solution was treated by extraction 3 times each with 200 ml of ether. The organic layers were combined, washed with a saturated aqueous solution of sodium chloride, dried over sodium sulfate and concentrated under a reduced pressure. The obtained (±)-1-methoxy-5-bis(4-biphenyl)methyl-bicyclo [3.3.0] -2-oxaoctane was used for the reaction in the next step without purification.

To a dry tetrahydrofuran solution (30 ml) containing (±)-1-methoxy-5-bis(4-biphenyl)methylbicyclo [3.3.0] -2-oxaoctane, 100 mg of active charcoal supporting 10% by weight of palladium was added. After the obtained suspension was stirred for 24 hours under the atmosphere of hydrogen of 100 kPa, the suspension was filtered. The separated solid substance was washed 3 times each with 100 ml of ether, and the organic layers were combined and concentrated under a reduced pressure. The obtained residue was purified in accordance with the silica gel column chromatography using a hexane/ethyl acetate mixed solvent (the ratio of the amounts by volume: 10/1) as the elution solvent, and 1.67 g (3.54 mmole) of (±)-1-methoxy-5-bis(4-cyclohexylphenyl)methylbicyclo-[3.3.0] -2-oxaoctane was obtained as a colorless liquid substance. The yield was 71%. The analytical data of the obtained compound are shown in the following.
FTIR (CHCl₃): 2923, 2850, 1510, 1447
¹H-NMR (400 MHz): 7.38 (d, J=7.6 Hz, 2H), 7.35 (d, J=8.0 Hz, 2H), 7.15 (d, J=7.6 Hz, 2H), 7.10 (d, J=8.0 Hz, 2H), 4.57 (s, 1H), 3.94-3.87 (m, 2H), 3.45 (s. 3H), 2.75 (dt, J=11.6, 7.6 Hz, 1H), 2.51-2.47 (m, 2H), 2.08-2.00 (m, 2H), 1.88-1.78 (m, 12H), 1.54-1.30 (m, 11H), 1.08-0.94 (m, 2H)
¹³C-NMR (100 MHz): 145.3 (C), 145.0 (C), 141.8 (C), 140.1 (C), 130.3 (CHx2), 1292 (CHx2), 126.2 (CHx2), 125.7 (CHx2), 117.9 (C), 65.6 (CH₂), 58.1 (C), 53.7 (CH), 50.5 (CH₃), 44.0 (CHx2), 40.0 (CH₂x2), 34.5 (CH₂x2), 34.4 (CH₂), 32.8 (CH₂), 31.9 (CH₂), 27.0 (CH₂x3), 26.2 (CH₂x3), 21.0 (CH₂)
HRMS (EI): Calcd. for C₃₃H₄₄O₂: 472.3341, Found: 472.3349

### Example 4

In accordance with the reaction route shown in scheme [12], (±)-1-methoxy-5-{4-(9-phenanthryl)phenyl}bicyclo [3.3.0] -2-oxaoctane was synthesized.

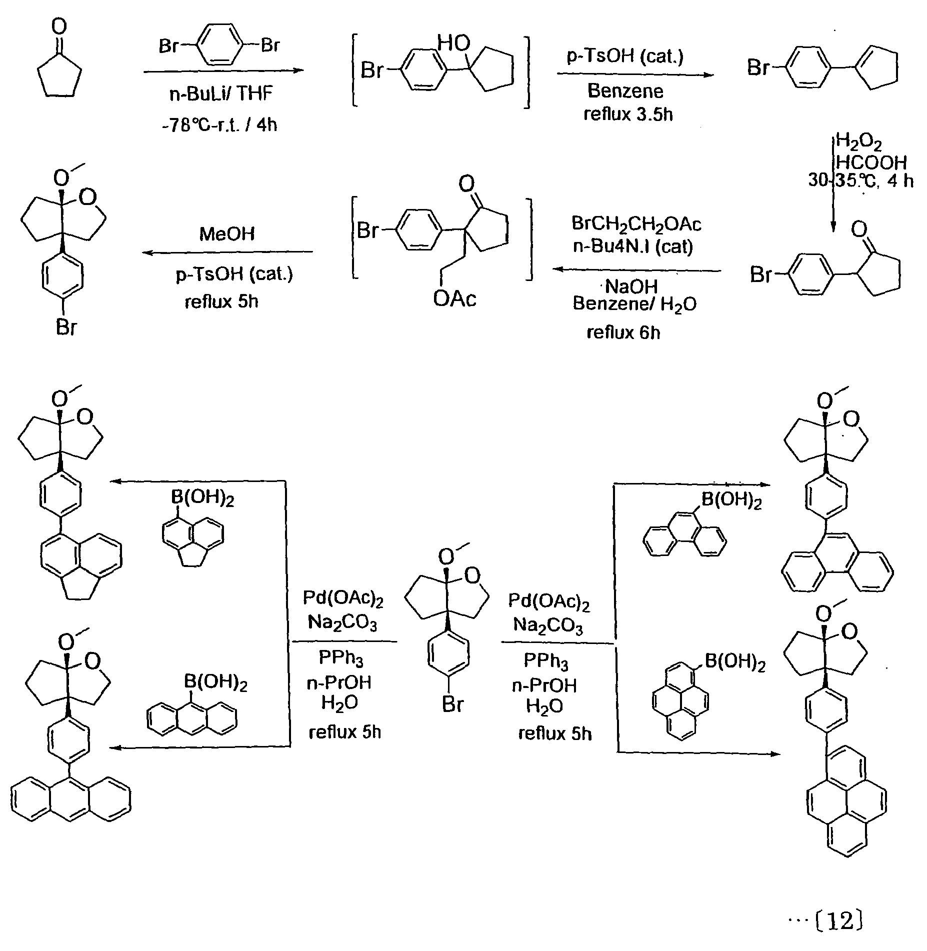

### (1) Synthesis of 4-bromo-1-cyclopenten-1-ylbenzene

To a dry tetrahydrofuran solution (900 ml) containing 88.5 g (0.38 moles) of 1,4-dibromobenzene, 146 ml (0.38 moles) of a 2.6 moles/liter hexane solution of butyllithium was added dropwise at -78°C. After the resultant solution was stirred for 1 hour, a dry tetrahydrofuran solution (100 ml) containing 33.6 g (0.4 moles) of cyclopentanone was added dropwise at -78°C. The temperature of the obtained reaction mixture was elevated to the room temperature over 12 hours. After 210 ml (0.42 moles) of a 2 moles/liter hydrochloric acid was added, the resultant mixture was treated by extraction 3 times each with 300 ml of ether. The organic layers were combined, washed with water and a saturated aqueous solution of sodium chloride, dried over magnesium sulfate and concentrated, and a light yellow liquid substance was obtained. The liquid substance was used for the reaction in the next step without purification.

To a benzene solution (500 ml) of the light yellow liquid substance, 1.72 g (9 mmole) of p-toluenesulfonic acid monohydrate was added, and the resultant mixture was heated under the refluxing condition for 3.5 hours and then concentrated under a reduced pressure. The obtained brown solid substance was purified in accordance with the silica gel column chromatography using hexane as the elution solvent, and 69.2 g (0.31 moles) of 4-bromo-1-cyclopenten-1-ylbenzene was obtained as colorless crystals. The yield was 82%. The analytical data of the obtained compound are shown in the following.
Melting point: 94-95°C (the solvent of recrystallization: hexane)
FTIR (CHCl₃): 3053, 2953, 2843, 1902, 1619, 1585, 1486
¹H-NMR (400 MHz): 7.41 (d, J=8.4 Hz, 2H), 7.27 (d, J=8.4 Hz, 2H), 6.17 (s, 1H), 2.68-2.63 (m, 2H), 2.53-2.48 (m, 2H), 2.01 (td, J=14.8, 7.6 Hz, 2H)
¹³C-NMR (100 MHz): 141.3 (C), 135.7 (C), 131.2 (CHx2), 127.1 (CHx2), 127.0 (CH), 120.5 (C), 33.5 (CH₂), 33.2 (CH₂), 23.4 (CH₂)
Anal. Calcd. for C₁₁H₁₁Br: C 59.22, H4.97; Found: C 58.91, H 4.94

### (2) Synthesis of (±)-2-(4-bromophenyl)cyclopentanone

A 30% by weight aqueous solution of hydrogen peroxide in an amount of 40 ml (0.39 moles, the specific gravity: 1.11) and 200 ml (5.25 moles, the specific gravity: 1.22) of a 99% by weight aqueous solution of formic acid were mixed at the room temperature, and the resultant mixture was stirred at 40 to 45°C for 30 minutes. To the obtained aqueous solution, an acetone solution (150 ml) containing 65 g (0.29 moles) of 4-bromo-1-cyclopenten-1-ylbenzene was added dropwise while the temperature was kept in the range of 30 to 35°C. After the addition was completed, the obtained mixture was stirred at 30°C for 4 hours. After water was removed by distillation at 45°C under 20 kPa, an aqueous solution of sodium hydroxide was added until the mixture became slightly alkaline. The obtained mixture was treated by extraction 3 times each with 300 ml of ether. The organic layers were combined, washed with water and a saturated aqueous solution of sodium chloride, dried over magnesium sulfate and concentrated under a reduced pressure. The obtained light yellow liquid substance was purified in accordance with the silica gel column chromatography using a hexane/ethyl acetate mixed solvent (the ratio of the amounts by volume: 9/1) as the elution solvent, and 31.3 g (0.132 moles) of (+)-2.(4.bromophenyl)cyclopentanone was obtained as a colorless liquid substance. The yield was 45%. The analytical data of the obtained compound are shown in the following.
FTIR (CHCl₃): 3029, 2966, 2876, 1899, 1741, 1589, 1488, 1402
¹H-NMR (400 MHz): 7.44 (d, J=8.4 Hz, 2H), 7.06 (d, J=8.4 Hz, 2H), 3.26 (dd, J=11.4, 8.0 Hz, 1H), 2.51-2.42 (m, 2H), 2.31-2.21 (m, 1H), 2.18-2.10 (m, 1H), 2.08-2.00 (m, 1H), 1.95-1.85 (m, 1H)
¹³C-NMR (100 MHz): 217.0 (C), 137.1 (C), 131.5 (CHx2), 129.7 (CHx2), 120.7 (C), 54.6 (CH), 38.2 (CH₂), 31.4 (CH₂), 20.8 (CH₂)

### (3) Synthesis of (±)-1-methoxy-5-(4-bromophenyl)bicyclo [3.3.0] -2-oxaoctane

To a benzene solution (300 ml) containing 31 g (0.13 moles) of (±)-2-(4-bromophenyl)cyclopentanone and 26 g (0.156 moles) of 2-bromoethyl acetate, 5 g (13.5 mmole) of tetrabutylammonium iodide was added at the room temperature, and the resultant mixture was stirred for 10 minutes. To the obtained suspension, 60 ml (0.65 moles) of a 10.8 moles/liter aqueous solution of sodium hydroxide was added, and the resultant mixture was heated under the refluxing condition for 6 hours and then cooled to the room temperature by leaving standing. To the obtained reaction mixture, 350 ml (0.70 moles) of a 2 moles/liter hydrochloric acid was added, and the obtained mixture was treated by extraction 3 times each with 250 ml of ether. The organic layers were combined, washed with water and a saturated aqueous solution of sodium chloride, dried over magnesium sulfate and concentrated under a reduced pressure, and a light brown liquid substance was obtained. The liquid substance was used for the reaction in the next step without purification.

To a methanol solution (300 ml) of the obtained light brown liquid substance, 0.86 g (0.45 mmole) of p-toluenesulfonic acid monohydrate was added at the room temperature, and the obtained mixture was heated under the refluxing condition for 5 hours. The reaction mixture was concentrated, and the obtained light brown liquid substance was purified in accordance with the silica gel column chromatography using a hexane/ethyl acetate mixed solvent (the ratio of the amounts by volume: 19/1) as the elution solvent, and 21.1 g (71 mmole) of (±)-1-methoxy-5-(4-bromophenyl)bicyclo [3.3.0] -2-oxaoctane was obtained as a colorless solid substance. The yield was 55%. The analytical data of the obtained compound are shown in the following.
The melting point: 48-49°C (the solvent of recrystallization: hexane)
FTIR (CHCl₃): 2955, 2829, 1489, 1316
¹H-NMR (400 MHz): 7.41 (d, J=8.8 Hz, 2H), 7.25 (d, J=8.8 Hz, 2H), 4.05 (t, J=7.2 Hz, 2H), 3.2 (s, 3H), 2.49-2.42 (m, 1H), 2.29-2.22 (m, 2H), 1.98-1.89 (m, 3H), 1.83-1.76 (m, 2H)
¹³C-NMR (100 MHz): 143.4 (C), 130.8 (CHx2), 129.1 (CHx2), 119.8 (C), 118.1 (C), 66.2 (CH₂), 58.6 (C), 50.9 (CH₃), 40.3 (CH₂), 38.1 (CH₂), 34.3 (CH₂), 21.4 (CH₃)
Anal. Calcd. for C₁₄H₁₇BrO₂: C 56.58, H, 5.77; Found: C 56.43, H 5.67

### (4) Synthesis of (±)-1-methoxy-5-{4-(9-phenanthryl)phenyl}bicyclo-[3.3.0] -2-oxaoctane

To n-propanol solution (25 ml) containing 2.0 g (6.73 mmole) of (±)-1-methoxy-5-(4-bromophenyl)bicyclo [3.3.0] -2-oxaoctane and 1.9 g (8.49 mmole) of 9-phenanthrylboronic acid, 0.015 g (0.065 mmole) of palladium(II) acetate and 0.052 g (0.198 mmole) of triphenylphosphine were added successively, and the resultant mixture was stirred at the room temperature for 30 minutes. To the obtained reaction mixture, 8 ml of a 2 moles/liter aqueous solution of sodium carbonate was added, and the resultant mixture was heated under the refluxing condition for 5 hours. To the obtained reaction mixture, water was added, and the resultant mixture was treated by extraction 3 times each with 50 ml of ether. The organic layers were combined, washed with water and a saturated aqueous solution of sodium chloride, dried over sodium sulfate and concentrated under a reduced pressure, and a light brown solid substance was obtained. The obtained solid substance was purified in accordance with the silica gel column chromatography using a hexane/ethyl acetate mixed solvent (the ratio of the amounts by volume: 9/1) as the elution solvent, and 2.0 g (4.96 mmole) of (±)-1-methoxy-5-{4-(9-phenanthryl)-phenyl}bicyclo [3.3.0] -2-oxaoctane was obtained as a colorless solid substance. The yield was 76%. The analytical data of the obtained compound are shown in the following.

The melting point: 144-145°C (the solvent of recrystallization: ethyl acetate/hexane)
FTIR (CHCl₃): 2950, 2881, 2828, 2244, 1610, 1510, 1492, 1450, 1317
¹H-NMR (400 MHz): 8.76 (d, J=8.0 Hz, 1H), 8.70 (d, J=8.0 Hz, 1H), 8.00 (d, J=8.0 Hz, 1H), 7.87 (d, J=8.0 Hz, 1H), 7.68-7.57 (m, 4H), 7.54-7.47 (m, 5H), 4.16-4.07 (m, 2H), 3.29 (s, 3H), 2.64-2.57 (m, 1H), 2.39-2.28 (m, 2H), 2.15-2.00 (m, 3H), 1.92-1.82 (m, 2H)
¹³C-NMR (100 MHz): 143.5 (C), 138.6 (C), 138.1 (C), 131.6 (C), 131.1 (C), 130.6 (C), 129.8 (C), 129.5 (CHx2), 128.6 (CH), 127.5 (CH), 127.2 (CHx2), 127.0 (CH), 126.7 (CH), 126.43 (CH), 126.36 (CHx2), 122.8 (CH), 122.5 (CH), 118.4 (C), 66.3 (CH₂), 58.9 (C), 51.0 (CH₃), 40.7 (CH₂), 38.2 (CH₂), 34.4 (CH₂), 21.5 (CH₂)
Anal. Calcd. for C₂₆H₂₆O₂: C 85.25, H, 6.64; Found: C 85.60, H 6.85

### Example 5

In accordance with the reaction route shown in scheme [12], (±)-1-methoxy-5-{4-(1-pyrenyl)phenyl}bicyclo [3.3.0] -2-oxaoctane was synthesized.

In accordance with the same procedures as those conducted in Example 4 except that 1-pyrenylboronic acid was used in place of 9-phenanthrylboronic acid, (±)-1-methoxy-5-{4-(1-pyrenyl)phenyl}bicyclo-[3.3.0] -2-oxaoctane was synthesized. As the elution solvent in the silica gel chromatography, a hexane/ethyl acetate mixed solvent (the ratio of the amounts by volume: 19/1) was used.

(±)-1-Methoxy-5-{4-(1-pyrenyl)phenyl}bicyclo [3.3.0] -2-oxaoctane in an amount of 1.80 g (4.30 mmole) was obtained as a colorless solid substance. The yield was 65%. The analytical data of the obtained compound are shown in the following.

The melting point: 196-197°C (the solvent of recrystallization: ethyl acetate/hexane)
FTIR (CHCl₃): 2955, 2828, 1917, 1796, 1601, 1584, 1520, 1499, 1402
¹H-NMR (400 MHz): 8.24 (d, J=9.2 Hz, 1H), 8.18 (d, J=8.0 Hz, 1H), 8.14 (t, J=8.0 Hz, 2H), 8.05 (s, 2H), 8.00-7.95 (m, 3H), 7.57-7.52 (m, 4H), 4.16-4.06 (m, 2H), 3.30 (s, 3H), 2.64-2.57 (m, 1H), 2.38-2.28 (m, 2H), 2.15-2.00 (m, 3H), 1.93-1.81 (m, 2H)
13C-NMR (100 MHz): 143.4 (C), 138.5 (C), 137.8 (C), 131.4 (C), 131.0 (C), 130.4 (C), 130.0 (CHx2), 128.4 (C), 127.6 (CH), 127.4 (CH), 127.28 (CHx3), 127.26 (CH), 127.24 (C), 125.9 (CH), 125.5 (CH), 124.95 (CH), 124.9 (C), 124.7 (CH), 124.6 (CH), 118.4 (C), 66.3 (CH₂), 58.9 (C), 51.0 (CH₃), 40.7 (CH₂), 38.2 (CH₂), 34.4 (CH₂), 21.5 (CH₂)
Anal. Calcd. for C₃₀H₂₆O₂: C 86.09, H 6.26; Found: C 85.93, H 6.37

### Example 6

In accordance with the reaction route shown in scheme [12], (±)-1-methoxy-5-{4-(5-acenaphthenyl)phenyl}bicyclo [3.3.0] -2-oxaoctane was synthesized.

In accordance with the same procedures as those conducted in Example 4 except that 5-acenaphthenylboronic acid was used in place of 9-phenanthrylboronic acid, (±)-1-methoxy-5-{4-(5-acenaphthenyl)phenyl}-bicyclo [3.3.0] -2-oxaoctane was synthesized.. As the elution solvent in the silica gel chromatography, a hexane/ethyl acetate mixed solvent (the ratio of the amounts by volume: 19/1) was used.

(±)-1-Methoxy-5-{4-(5-acenaphthenyl)phenyl}bicyclo [3.3.0] -2-oxaoctane in an amount of 1.84 g (4.97 mmole) was obtained as a colorless solid substance. The yield was 74%. The analytical data of the obtained compound are shown in the following.

The melting point: 155-156°C (the solvent of recrystallization: ethyl acetate/hexane)

FTIR (CHCl₃): 3027, 2948, 2880, 2829, 1605, 1515, 1495, 1447, 1426, 1366
¹H-NMR (400 MHz): 7.76 (d, J=8.0 Hz, 1H), 7.52-7.47 (m, 4H), 7.43-7.38 (m, 2H), 7.35-7.25 (m, 2H), 4.15-4.06 (m, 2H), 3.41 (s, 4H), 3.27 (s, 3H), 2.62-2.55 (m, 1H), 2.37-2.27 (m, 2H), 2.13-1.98 (m, 3H), 1.94-1.80 (m, 2H)
¹³C-NMR (100 MHz): 146.0 (C), 145.3 (C), 143.1 (C), 139.5 (C), 137.4 (C), 135.4 (C), 129.7 (C), 129.1 (CHx2), 128.5 (CH), 127.8 (CH), 127.2 (CHx2), 121.0 (C), 119.2 (CH), 119.1 (CH), 118.4 (CH), 66.2 (CH₂), 58.8 (C), 50.9 (CH₃), 40.7 (CH₂), 38.1 (CH₂), 34.4 (CH₂), 30.6 (CH₂), 30.1 (CH₂), 21.5 (CH₂)
Anal. Calcd. for C₂₆H₂₆O₂: C 84.29, H 7.07; Found: C 84.56, H 7.21

### Example 7

In accordance with the reaction route shown in scheme [12], (±)-1-methoxy-5-{4-(9-anthryl)phenyl}bicyclo [3.3.0] -2-oxaoctane was synthesized.

In accordance with the same procedures as those conducted in Example 4 except that 9-anthrylboronic acid was used in place of 9-phenanthrylboronic acid, (±)-1-methoxy-5-{4-(9-anthryl)phenyl}bicyclo-[3.3.0] -2-oxaoctane was synthesized.. As the elution solvent in the silica gel chromatography, a hexane/ethyl acetate mixed solvent (the ratio of the amounts by volume: 19/1) was used.

(±)-1-Methoxy-5-{4-(9-anthryl)phenyl}bicyclo [3.3.0] -2-oxaoctane in an amount of 0.97 g (2.46 mmole) was obtained as a colorless solid substance. The yield was 37%. The analytical data of the obtained compound are shown in the following.

The melting point: 225-226°C (the solvent of recrystallization: ethyl acetate/hexane)
FTIR (CHCl₃): 3048, 2971, 2886, 2830, 1815, 1621, 1514, 1441, 1412, 1362, 1319
¹H-NMR (400 MHz): 8.48 (s, 1H), 8.03 (d, J=8.4 Hz, 2H), 7.73 (d, J=8.0 Hz, 2H), 7.58 (t, J=7.6 Hz, 2H), 7.45 (d, J=6.8 Hz, 2H), 7.37 (d, J=8.0 Hz, 2H), 7.34 (t, J=7.6 Hz, 2H), 4.21-4.11 (m, 2H), 3.32 (s, 3H), 2.71-2.63 (m, 1H), 2.46-2.40 (m, 1H), 2.38-2.31 (m, 1H), 2.22-2.04 (m, 3H), 2.00-1.85 (m, 2H)
¹³C-NMR (100 MHz): 143.7 (C), 137.0 (C), 135.9 (C), 131.4 (Cx2), 130.7 (CHx2), 130.3 (Cx2), 128.2 (CHx2), 127.2 (CHx2), 127.0 (CHx2), 126.4 (CH), 125.1 (CHx2), 125.0 (CHx2), 118.5 (C), 66.4 (CH₂), 59.0 (C), 51.0 (CH₃), 41.1 (CH₂), 38.2 (CH₂), 34.7 (CH₂), 21.6 (CH₂)
Anal. Calcd. for C₂₈H₂₆O₂: C 85.25, H 6.64; Found: C 85.20, H 6.77

### Example 8

Using (+)-1-methoxy-5-fluorenylidenemethylbicyclo [3.3.0] -2-oxaoctane obtained in Example 1 as the agent for optical resolution, optical resolution of racemic 2-heptanol was conducted.

In a reactor equipped with a reflux condenser, 36.8 parts by weight of toluene, 1.16 parts by weight of racemic 2-heptanol and 3.68 parts by weight of molecular sieves 4A were placed, and 72.8 parts by weight of molecular sieves 4A for adsorbing methanol was packed into the reflux condenser. At the room temperature, 3.18 parts by weight of (+)-1-methoxy-5-fluorenylidenemethylbicyclo [3.3.0] -2-oxaoctane was added, and the reaction was allowed to proceed by heating for 7 hours under the refluxing condition.

After the reaction was completed, the reaction mixture was filtered. The filtrate was concentrated under a reduced pressure, and a mixture of diastereomers containing (R)-1-(1-methylhexyloxy)-5-fluorenylidenemethylbicyclo [3.3.0] -2-oxaoctane and (S)-1-(1-methylhexyloxy)-5-fluorenylidenemethylbicyclo [3.3.0] -2-oxaoctane was obtained as a yellow oily substance.

The mixture of the diastereomers was resolved into each component in accordance with the thin layer chromatography using a plate for the silica gel thin layer chromatography [manufactured by MERCK Company; KIESELGEL 60F256; a silica gel TLC plate] and a hexane/toluene mixed solvent (the ratio of the amounts by volume: 1/1) as the developing solvent. The difference in the rate of flow ΔRf was 0.111.

### Examples 9 to 11

In accordance with the same procedures as those conducted in Example 8 except that racemic 2-octanol, 2-undecanol or 2-pentadecanol was used in place of racemic 2-heptanol, a mixture of diastereomers was synthesized by bringing the racemic compound and (+)-1-methoxy-5-fluorenylidenemethylbicyclo [3.3.0] -2-oxaoctane into reaction with each other and was resolved in accordance with the thin layer chromatography. The reaction was conducted using 3.18 parts by weight of (+)-1-methoxy-5-fluorenylidenemethylbicyclo [3.3.0] -2-oxaoctane and the same amount by mole of the racemic compound.

The difference in the rate of flow ΔRf was 0.136 for 2-octanol, 0.146 for 2-undecanol and 0.156 for 2-pentadecanol.

### Examples 12 and 13

In accordance with the same procedures as those conducted in Example 8 except that racemic 1-(2-naphthyl)hexanol or 1-(2-naphthyl)-decanol was used in place of racemic 2-heptanol, a mixture of diastereomers was synthesized by bringing the racemic compound and (+)-1-methoxy-5-fluorenylidenemethylbicyclo [3.3.0] -2-oxaoctane into reaction with each other and was resolved in accordance with the thin layer chromatography. The reaction was conducted using 3.18 parts by weight of (+)-1-methoxy-5-fluorenylidenemethylbicyclo [3.3.0] -2-oxaoctane and the same amount by mole of the racemic compound.

The difference in the rate of flow ΔRf was 0.108 for 1-(2-naphthyl)-hexanol and 0.136 for 1-(2-naphthyl)decanol.

### Examples 14 to 17

In accordance with the same procedures as those conducted in Example 8 except that racemic 1-phenylhexanol, 1-(2-fluorophenyl)-hexanol, 1-(2-furyl)hexanol or 1-(2-naphthyl)hexanol was used in place of racemic 2-heptanol, a mixture of diastereomers was synthesized by bringing the racemic compound and (+)-1-methoxy-5-fluorenylidenemethylbicyclo [3.3.0] -2-oxaoctane into reaction with each other and was resolved in accordance with the thin layer chromatography. The reaction was conducted using 3.18 parts by weight of (+)-1-methoxy-5-fluorenylidenemethylbicyclo [3.3.0] -2-oxaoctane and the same amount by mole of the racemic compound.

The difference in the rate of flow ΔRf was 0.106 for 1-phenylhexanol, 0.133 for 1-(2-fluorophenyl)hexanol, 0.125 for 1-(2-furyl)hexanol and 0.108 for 1-(2-naphthyl)hexanol.

### Example 18

In accordance with the same procedures as those conducted in Example 8 except that 1.18 parts by weight of racemic ethyl lactate was used in place of racemic 2-heptanol, a mixture of diastereomers was synthesized by bringing the racemic compound and (+)-1-methoxy-5-fluorenylidenemethylbicyclo [3.3.0] -2-oxaoctane into reaction with each other and was resolved in accordance with the thin layer chromatography using a hexane/ethyl acetate mixed solvent (the ratio of the amounts by volume: 5/1) as the developing solvent.

The difference in the rate of flow ΔRf was 0.102.

### Example 19

In accordance with the same procedures as those conducted in Example 8 except that 1.14 parts by weight of racemic 1-vinylpentanol was used in place of racemic 2-heptanol, a mixture of diastereomers was synthesized by bringing the racemic compound and (+)-1-methoxy-5-fluorenylidenemethylbicyclo [3.3.0] -2-oxaoctane into reaction with each other and was resolved in accordance with the thin layer chromatography

The difference in the rate of flow ΔRf was 0.156.

The results in Examples 8 to 19 are shown in Table 1.

**Table 1**

| | Agent for optical resolution | Racemic compound | ΔRf |
|---|---|---|---|
| Example 8 | (+)-1-methoxy- | 2-heptanol | 0.111 |
| Example 9 | 5-fluorenylidene- | 2-octanol | 0.136 |
| Example 10 | methylbicyclo- | 2-undecanol | 0.146 |
| Example 11 | [3.3.0] -2-oxaoctane | 2-pentadecanol | 0.156 |
| Example 12 | in all Examples | 1-(2-naphthyl)hexanol | 0.108 |
| Example 13 | | 1-(2-naphthyl)decanol | 0.136 |
| Example 14 | | 1-phenylhexanol | 0.106 |
| Example 15 | | 1-(2-fluorophenyl)hexanol | 0.133 |
| Example 16 | | 1-(2-furyl)hexanol | 0.125 |
| Example 17 | | 1-(2-naphthyl)hexanol | 0.108 |
| Example 18 | | ethyl lactate | 0.102 |
| Example 19 | | 1-vinylpentanol | 0.156 |

As shown in Table 1, the mixture of diastereomers obtained from a racemic alcohol and (+)-1-methoxy-5-fluorenylidenemethylbicyclo [3.3.0] -2- oxaoctane provided a great difference in the rate of flow ΔRf in the thin layer chromatography. Thus, it is shown that (+)-1-methoxy-5-fluorenylidenemethylbicyclo [3.3.0] -2-oxaoctane exhibited the excellent ability as the agent for optical resolution.

### Example 20

Optical resolution was conducted using a mixture of diastereomers obtained from the reaction of (±)-1-methoxy-5-bis(4-cyclohexylphenyl)-methylbicyclo [3.3.0] -2-oxaoctane obtained in Example 3 and (R)-2-octanol.

In a reactor equipped with a reflux condenser, 36.8 parts by weight of toluene, 1.30 parts by weight of (R)-2-octanol and 3.68 parts by weight of molecular sieves 5A were placed, and 72.8 parts by weight of molecular sieves 4A for adsorbing methanol was packed into the reflux condenser. At the room temperature, 4.72 parts by weight of (±)-1-methoxy-5-bis(4-cyclohexylphenyl)methylbicyclo [3.3.0] -2-oxaoctane was added, and the reaction was allowed to proceed by heating for 7 hours under the refluxing condition to obtain a mixture of diastereomers.

The mixture of diastereomers was resolved in accordance with the thin layer chromatography using a plate for the silica gel thin layer chromatography [manufactured by MERCK Company; KIESELGEL 60F256; a silica gel TLC plate] and a hexane/toluene mixed solvent (the ratio of the amounts by volume: 1/1) as the developing solvent. The difference in the rate of flow ΔRf was 0.102.

### Example 21

In accordance with the same procedures as those conducted in Example 20, optical resolution was conducted using a mixture of diastereomers obtained by the reaction of (±)-1-methoxy-5-{4-(9-phenanthryl)phenyl}bicyclo [3.3.0] -2-oxaoctane obtained in Example 4 with (R)-2-octanol. The amount of (±)-1-methoxy-5-{4-(9-phenanthryl)-phenyl}bicyclo [3.3.0] -2-oxaoctane was 3.94 parts by weight. As the developing solvent, a hexane/toluene mixed solvent (the ratio of the amounts by volume: 1/2) was used.

The difference in the rate of flow ΔRf was 0.121.

### Examples 22 and 23

In accordance with the same procedures as those conducted in Example 20, optical resolution was conducted using a mixture of diastereomers obtained by the reaction of (±)-1-methoxy-5-{4-(9-phenanthryl)phenyl}bicyclo [3.3.0] -2-oxaoctane obtained in Example 4 with (R)-2-heptanol or (R)-1-cyclopentylethanol. The amount of (±)-1-methoxy-5-{4-(9-phenanthryl)phenyl}bicyclo [3.3.0] -2-oxaoctane was 3.94 parts by weight, and the same amount by mole of (R)-2-heptanol or (R)-1-cyclopentylethanol was used. As the developing solvent, a hexane/toluene mixed solvent (the ratio of the amounts by volume: 1/4) was used.

The difference in the rate of flow ΔRf was 0.115 for (R)-2-heptanol and 0.121 for (R)-1-cyclopentylethanol.

### Example 24

In accordance with the same procedures as those conducted in Example 20, optical resolution was conducted using a mixture of diastereomers obtained by the reaction of (±)-1-methoxy-5-{3-(9-phenanthryl)phenyl}bicyclo [3.3.0] -2-oxaoctane with (R)-2-octanol. The amount of (±)-1-methoxy-5-{3-(9-phenanthryl)phenyl}bicyclo [3.3.0] -2-oxaoctane was 3.94 parts by weight.

The difference in the rate of flow ΔRf was 0.123.

### Example 25

In accordance with the same procedures as those conducted in Example 20, optical resolution was conducted using a mixture of diastereomers obtained by the reaction of (±)-1-methoxy-5-{4-(1-pyrenyl)-phenyl}bicyclo [3.3.0] -2-oxaoctane obtained in Example 5 with (R)-2-octanol. The amount of (±)-1-methoxy-5-{4-(1-pyrenyl)phenyl}bicyclo-[3.3.0] -2-oxaoctane was 4.18 parts by weight.

The difference in the rate of flow ΔRf was 0.113.

### Example 26

In accordance with the same procedures as those conducted in Example 20, optical resolution was conducted using a mixture of diastereomers obtained by the reaction of (±)-1-methoxy-5-{4-(5-acenaphthenyl)phenyl}bicyclo [3.3.0] -2-oxaoctane obtained in Example 6 with (R)-2-octanol. The amount of (±)-1-methoxy-5-{4-(5-acenaphthenyl)phenyl}bicyclo [3.3.0] -2-oxaoctane was 3.70 parts by weight. As the developing solvent, a hexane/toluene mixed solvent (the ratio of the amounts by volume: 5/4) was used.

The difference in the rate of flow ΔRf was 0.083.

### Example 27

In accordance with the same procedures as those conducted in Example 20, optical resolution was conducted using a mixture of diastereomers obtained by the reaction of (±)-1-methoxy-5-{4-(9-anthryl)phenyl}bicyclo [3.3.0] -2-oxaoctane obtained in Example 7 with (R)-2-octanol. The amount of (±)-1-methoxy-5-{4-(9-anthryl)-phenyl}bicyclo [3.3.0] -2-oxaoctane was 3.94 parts by weight. As the developing solvent, a hexane/toluene mixed solvent (the ratio of the amounts by volume: 5/4) was used.

The difference in the rate of flow ΔRf was 0.110.

### Example 28

In accordance with the same procedures as those conducted in Example 20, optical resolution was conducted using a mixture of diastereomers obtained by the reaction of (±)-1-methoxy-5-{4-(9-fluorenyl)phenyl}bicyclo [3.3.0] -2-oxaoctane with (R)-2-octanol. The amount of (±)-1-methoxy-5-{4-(9-fluorenyl)phenyl}bicyclo [3.3.0] -2-oxaoctane was 3.82 parts by weight. As the developing solvent, a hexane/toluene mixed solvent (the ratio of the amounts by volume: 1/4) was used.

The difference in the rate of flow ΔRf was 0.119.

### Example 29

In accordance with the same procedures as those conducted in Example 20, optical resolution was conducted using a mixture of diastereomers obtained by the reaction of (±)-1-methoxy-5-(2-naphthyl)-bicyclo [3.3.0] -2-oxaoctane with (R)-2-octanol. The amount of (±)-1-methoxy-5-(2-naphthyl)bicyclo [3.3.0] -2-oxaoctane was 2.68 parts by weight.

The difference in the rate of flow ΔRf was 0.119.

### Comparative Example 1

In accordance with the same procedures as those conducted in Example 20, optical resolution was conducted using a mixture of diastereomers obtained by the reaction of (±)-1-methoxy-5-(4-bromophenyl)bicyclo [3.3.0] -2-oxaoctane obtained as an intermediate compound in Example 4 with (R)-2-octanol. The amount of (±)-1-methoxy-5-(4-bromophenyl)bicyclo [3.3.0] -2-oxaoctane was 2.97 parts by weight.

The difference in the rate of flow ΔRf was 0.025.

The results in Example 20 to 29 and Comparative Example 1 are shown in Table 2.

**Table 2**

| | Substituent at 5-position | (R)-Compound | ΔRf |
|---|---|---|---|
| Example 20 | bis(4-cyclohexylphenyl)-methyl | 2-octanol | 0.102 |
| Example 21 | 4-(9-phenanthryl)phenyl | 2-octanol | 0.121 |
| Example 22 | 4-(9-phenanthryl)phenyl | 2-heptanol | 0.115 |
| Example 23 | 4-(9-phenanthryl)phenyl | 1-cyclopentyl-ethanol | 0.121 |
| Example 24 | 3-(9-phenanthryl)phenyl | 2-octanol | 0.123 |
| Example 25 | 4-(1-pyrenyl)phenyl | 2-octanol | 0.113 |
| Example 26 | 4-(5-acenaphthenyl)phenyl | 2-octanol | 0.083 |
| Example 27 | 4-(9-anthryl)phenyl | 2-octanol | 0.110 |
| Example 28 | 4-(9-fluorenyl)phenyl | 2-octanol | 0.119 |
| Example 29 | 2-naphthyl | 2-octanol | 0.119 |
| Comparative Example 1 | 4-bromophenyl | 2-octanol | 0.025 |

As shown in Table 2, the mixture of diastereomers obtained from the (±)-1-methoxybicyclo [3.3.0] -2-oxaoctane derivative having a condensed polycyclic hydrocarbon group or a group having at least 3 cyclic structures at the 5-position and an alcohol of the (R)-compound provided a great difference in the rate of flow ΔRf in the thin layer chromatography, and it is shown that, when the (±)-1-methoxybicyclo [3.3.0] -2-oxaoctane derivatives were resolved into the (+)-compound and the (-) compound, these compounds can work as the agent for optical resolution exhibiting the excellent ability.

In contrast, the mixture of diastereomers obtained from the (±)-1-methoxy-5-(4-bromophenyl)bicyclo [3.3.0] -2-oxaoctane having 4-bromophenyl group at the 5-position and an alcohol of the (R)-compound provided a small difference in the rate of flow ΔRf in the thin layer chromatography, and it is considered that, even when the (±)-1-methoxy-5-(4-bromophenyl)bicyclo [3.3.0] -2- oxaoctane are resolved into the (+)-compound and the (-) compound, these compounds can not work as the agent for optical resolution exhibiting the excellent ability.

### INDUSTRIAL APPLICABILITY

When the agent for optical resolution and the process for producing an optically active substance of the present invention are used, the mixture of diastereomers obtained by the reaction of the 1-alkoxybicyclo [3.3.0] -2-oxaoctane compound represented by the formula [1], the 1-hydroxybicyclo [3.3.0] -2-oxaoctane compound represented by the formula [2] or the bicyclo [3.3.0] -2-oxa-1-octene compound represented by the formula [3] and a mixture of optically active substances having active hydrogen atom can be resolved well, so that the optically active substance having a very high optical purity can be obtained by decomposing the diastereomer obtained by the resolution. The 1-alkoxybicyclo [3.3.0] -2-oxaoctane compound represented by the formula [1] or the 1-hydroxybicyclo [3.3.0] -2-oxaoctene compound represented by the formula [2] which are obtained by decomposition of the diastereomer can be used repeatedly as the agent for optical resolution.

## Claims

1. An agent for optical resolution which comprises:
a 1-alkoxybicyclo [3.3.0] -2-oxaoctane compound represented by the formula [1],
a 1-hydroxybicyclo [3.3.0] -2-oxaoctane compound represented by the formula [2] or
a bicyclo [3.3.0] -2-oxa-1-octene compound represented by the formula [3] : 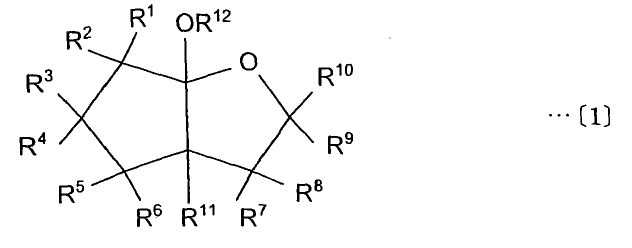 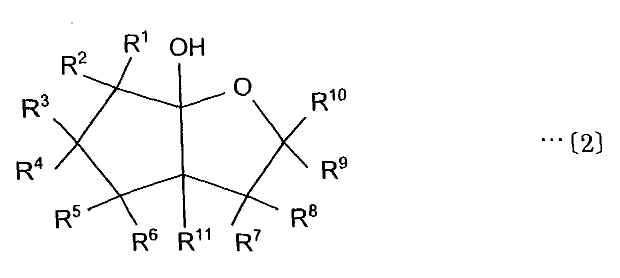 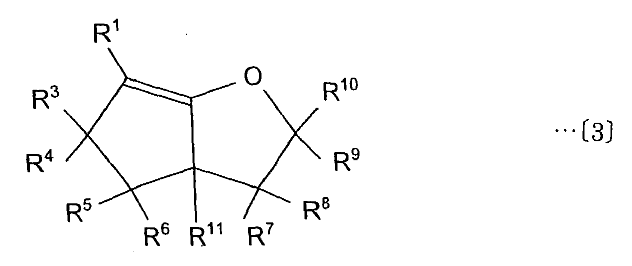
wherein R¹ to R¹⁰ each independently represents hydrogen atom or an alkyl group having 1 to 20 carbon atoms, R¹¹ represents a condensed polycyclic hydrocarbon group or a group having at least three cyclic structures, and R¹² represents an alkyl group having 1 to 6 carbon atoms.

2. A process for producing an optically active substance which comprises the steps of:
bringing a 1-alkoxybicyclo [3.3.0] -2-oxaoctane compound represented by the formula [1], a 1-hydroxybicyclo [3.3.0] -2-oxaoctane compound represented by the formula [2] or a bicyclo [3.3.0] -2-oxa-1-octene compound represented by the formula [3], into reaction with a mixture of optically active substances having active hydrogen atom to form a mixture of diastereomers;
resolving the mixture of the diastereomers into each diastereomer; and
decomposing at least one of the diastereomers obtained by resolution to obtain an (R) optically active substance or an (S) optically active substance: 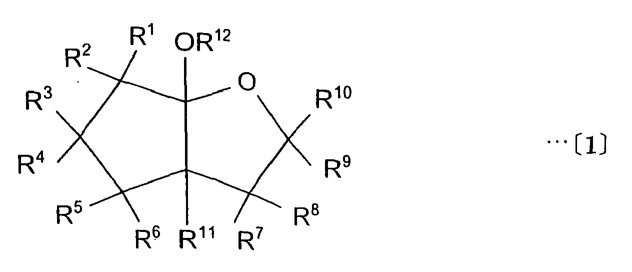 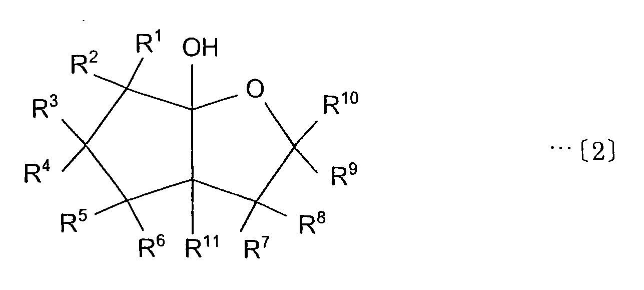 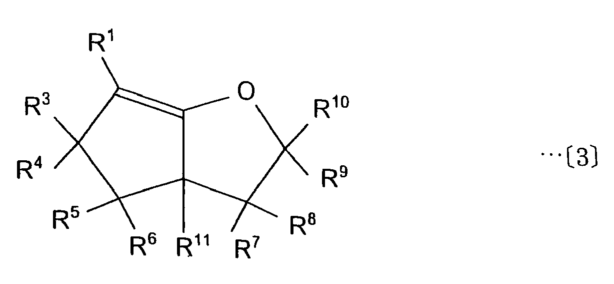
wherein R¹ to R¹⁰ each independently represents hydrogen atom or an alkyl group having 1 to 20 carbon atoms, R¹¹ represents a condensed polycyclic hydrocarbon group or a group having at least three cyclic structures, and R¹² represents an alkyl group having 1 to 6 carbon atoms.

3. A 1-alkoxybicyclo [3.3.0] -2-oxaoctane compound represented by the formula [1], a 1-hydroxybicyclo [3.3.0] -2-oxaoctane compound represented by the formula [2] or a bicyclo [3.3.0] -2-oxa-1-octene compound represented by the formula [3] : 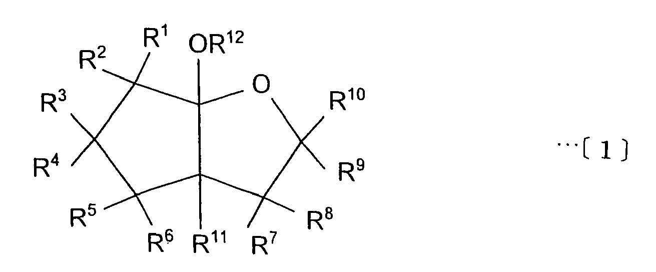 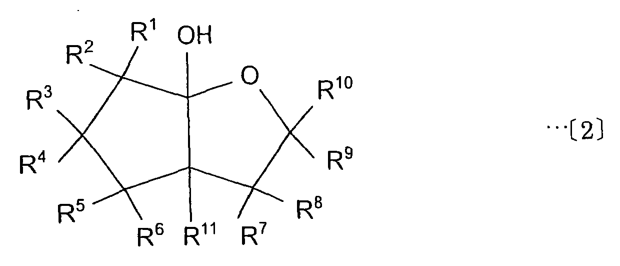 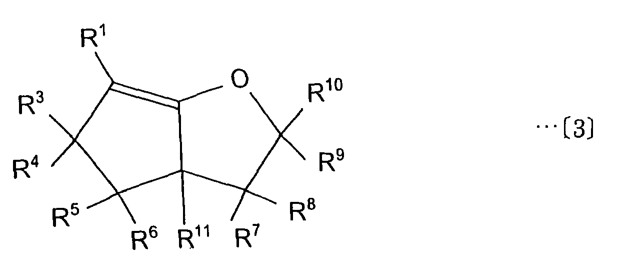 wherein R¹ to R¹⁰ each independently represents hydrogen atom or an alkyl group having 1 to 20 carbon atoms, R¹¹ represents fluorenylmethyl group or fluorenylidenemethyl group, and R¹² represents an alkyl group having 1 to 6 carbon atoms.

4. A 1-methoxybicyclo [3.3.0] -2-oxaoctane compound represented by the formula [4] : 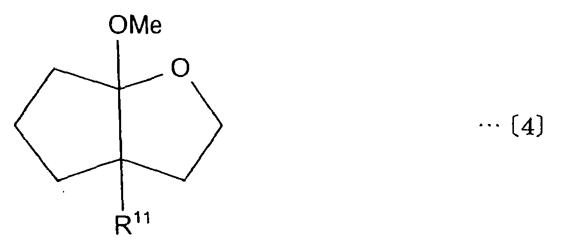 wherein R¹¹ represents bis(4-cyclohexylphenyl)methyl group, 4-(9-phenanthryl)phenyl group, 4-(1-pyrenyl)phenyl group, 4-(5-acenaphthenyl)phenyl group or 4-(9-anthryl)phenyl group.
